(19) **Europäisches Patentamt / European Patent Office / Office européen des brevets**

(11) **EP 4 351 417 B1**

(12) **EUROPEAN PATENT SPECIFICATION**

(45) Date of publication and mention
of the grant of the patent:
**01.04.2026 Bulletin 2026/14**

(21) Application number: **22738814.7**

(22) Date of filing: **09.06.2022**

(51) International Patent Classification (IPC):
*A61B 5/113* (2006.01)   *A61B 5/00* (2006.01)
*A61N 1/36* (2006.01)   *A61B 5/08* (2006.01)

(52) Cooperative Patent Classification (CPC):
**A61B 5/1135; A61B 5/0816; A61B 5/4818;
A61B 5/4836; A61B 5/686; A61N 1/36078;
A61N 1/36139;** A61N 1/3611

(86) International application number:
**PCT/US2022/032821**

(87) International publication number:
**WO 2022/261311 (15.12.2022 Gazette 2022/50)**

(54) **RESPIRATION SENSING**

ATMUNGSMESSUNG

DÉTECTION DE RESPIRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.06.2021 US 202163209150 P**

(43) Date of publication of application:
**17.04.2024 Bulletin 2024/16**

(73) Proprietor: **Inspire Medical Systems, Inc.
Golden Valley, MN 55416 (US)**

(72) Inventors:
• **THORP, Christopher
Golden Valley, Minnesota 55416 (US)**
• **DIEKEN, David
Golden Valley, Minnesota 55416 (US)**
• **NESBITT, Nicholas
Golden Valley, Minnesota 55416 (US)**
• **RONDONI, John
Golden Valley, Minnesota 55416 (US)**

• **MANNE-NICHOLAS, James
Golden Valley, Minnesota 55416 (US)**
• **JONES, Ross Peter
Cambridge CB1 8QH (GB)**
• **SCHATZA, Mark
Golden Valley, Minnesota 55416 (US)**
• **WARWICK, Christopher Nicholas
Royston Hertfordshire SG8 7EU (GB)**
• **STONE, Jonathan Tudor
Swindon Wiltshire SN2 1SF (GB)**
• **CANAS DE MATOS E OLIVEIRA RIBEIRO,
Frederico
Cambridge CB2 9LS (GB)**

(74) Representative: **Zimmermann & Partner
Patentanwälte mbB
Postfach 330 920
80069 München (DE)**

(56) References cited:
**WO-A1-2021/016558     WO-A1-2021/016562
US-A- 5 522 862     US-A1- 2019 009 093**

## Description

Background

A significant portion of the population suffers from various forms of sleep disordered breathing (SDB). In some patients, external breathing therapy devices and/or mere surgical interventions may fail to treat the sleep disordered breathing behavior.

Brief Description of the Drawings

FIG. 1A is a block diagram illustrating one example of a device for detecting respiration.
FIG. 1B is a block diagram illustrating one example of a device for detecting respiration and applying stimulation based on the detected respiration.
FIG. 2 is a diagram including a side view, schematically representing an example method and/or example device for detecting respiration via an acceleration sensor.
FIG. 3 is a diagram, including a side view, schematically representing an example method and/or example device for detecting respiration via an acceleration sensor at a chest wall.
FIG. 4 is a diagram including a graph schematically representing an example filtered, sensed acceleration signal.
FIGS. 5A-5D are diagrams including graphs schematically representing example filtered, sensed acceleration signals for two different sensor orientations.
FIGS. 5E and 5F are diagrams including graphs schematically representing reference signals for fitting to example filtered, sensed acceleration signals.
FIG. 5G is a diagram including a graph schematically representing fitting example peaks and troughs of a filtered, sensed acceleration signal to reference signals.
FIG. 5H is a diagram including a graph schematically representing an example filtered, sensed acceleration signal and the estimated rate-of-change of the example filtered, sensed acceleration signal.
FIG. 5I is a diagram including a graph schematically representing a locus of the estimated rate-of-change of the example filtered, sensed acceleration signal versus the example filtered, sensed acceleration signal of FIG. 5H.
FIG. 6A is a flow diagram illustrating one example of a method for identifying the slope of an expiratory phase.
FIG. 6B is a flow diagram illustrating one example of a method for identifying the slope of an inspiratory phase.
FIGS. 7A and 7B are each a diagram, including a side view, schematically representing an example method and/or example device for detecting respiration via an acceleration sensor.
FIG. 8 is a diagram schematically representing example acceleration sensing elements.
FIG. 9A is a diagram including a side view schematically representing an example method and/or example device for detecting respiration with a patient relative to an angled support.
FIGS. 9B and 9C are each a diagram schematically representing an example method and/or example device including a sensing element extending at a particular angle relative to a gravity vector.
FIG. 10 is a diagram including a side view schematically representing an example method and/or example device for detecting respiration with a patient relative to an upright support.
FIG. 11 is a diagram including a front view schematically representing an example method and/or example device in which different sensing elements of an acceleration sensor are oriented relative to a patient's body.
FIG. 12 is a diagram including a side view schematically representing an example method and/or example device in which different sensing elements of an acceleration sensor are oriented relative to a patient's body.
FIG. 13 is a diagram including a front view schematically representing an example method and/or example device including an implantable medical device comprising an acceleration sensor.
FIG. 14A is a diagram schematically representing an example method of arranging an acceleration sensor.
FIG. 14B is a diagram schematically representing an example method of identifying a sensing element in relation to a reference angular orientation.
FIG. 14C is a diagram schematically representing an example method of determining a reference angular orientation.
FIG. 14D is a diagram schematically representing an example method of implementing sensing.
FIG. 14E is a diagram schematically representing an example method of determining respiration information via an identified sensing element.
FIG. 14F is a diagram schematically representing an example method of sensing within a range of angular orientations.
FIG. 14G is a diagram schematically representing an example method of identifying which sensing element exhibits a reference angular orientation.

FIG. 14H is a diagram schematically representing an example method of determining respiration information using an identified sensing element in relation to a greatest range of angular orientations.

FIG. 14I is a diagram schematically representing an example method of identifying a sensing element in relation to a greatest range of values of an AC signal component.

FIG. 14J is a diagram schematically representing an example method of determining respiration information using an identified sensing element in relation to a greatest range of values of an AC signal component.

FIG. 14K is a diagram schematically representing an example method of sensing an AC signal component during breathing.

FIG. 15 is a diagram including a front view schematically representing an example method and/or example device including a medical device, including an accelerometer, implanted in a patient's chest.

FIG. 16A is a block diagram schematically representing an example method and/or example device for determining respiration information via a sensed acceleration signal.

FIG. 16B is a block diagram schematically representing an example confidence factor portion.

FIG. 16C is a block diagram schematically representing an example feature extraction portion.

FIG. 16D is a block diagram schematically representing an example inspiratory phase prediction function.

FIG. 16E is a block diagram schematically representing an example noise model parameter.

FIG. 16F is a block diagram schematically representing another example method and/or example device for determining respiration information via a sensed acceleration signal.

FIGS. 17A and 17B each are a block diagram schematically representing an example control portion.

FIG. 18 is a block diagram schematically representing an example user interface.

FIG. 19 is a flow diagram illustrating one example of a method of treating sleep disordered breathing.

FIG. 20 is a flow diagram illustrating another example of a method of treating sleep disordered breathing.

Detailed Description

In the following detailed description, reference is made to the accompanying drawings which form a part hereof, and in which is shown by way of illustration specific examples in which the disclosure may be practiced. It is to be understood that other examples may be utilized and structural or logical changes may be made without departing from the scope of the present disclosure. The following detailed description, therefore, is not to be taken in a limiting sense. It is to be understood that features of the various examples described herein may be combined, in part or whole, with each other, unless specifically noted otherwise. The invention is defined by the appended claims.

At least some examples of the present disclosure are directed to detecting respiration information. In some such examples, the detection of respiration may be based, at least in part, on sensing rotational movement at a respiratory body portion caused by breathing and/or attempted breathing. In some examples, the respiratory body portion may comprise a chest (e.g., chest wall), abdomen (e.g., abdominal wall), and/or other body portion exhibiting rotational movement indicative of respiration or attempted respiration. Throughout the present disclosure, references to breathing and respiration also include, in at least some examples, attempted breathing and attempted respiration, respectively.

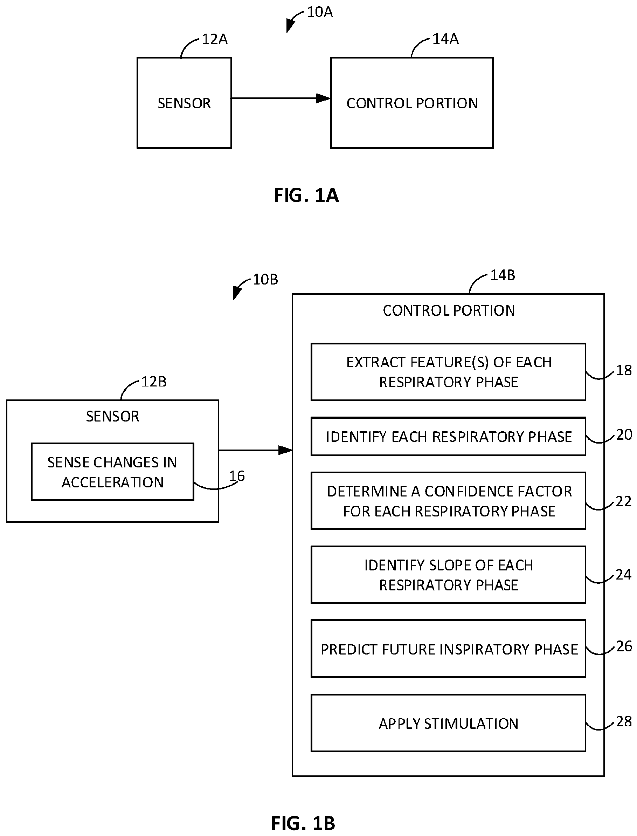

FIG. 1A is a block diagram illustrating one example of a device 10A for detecting respiration. Device 10A includes a sensor 12A and a control portion 14A electrically coupled to the sensor 12A. The sensor 12A is securable to a patient to provide a sensor signal indicative of respiration of the patient. In one example, sensor 12A includes an implantable accelerometer to sense changes in acceleration indicative of respiration of the patient. The control portion 14A may extract features from the sensor signal to determine each respiratory phase of the patient. In one example, the control portion 14A determines a midpoint of each respiratory phase (e.g., inspiratory phase and/or expiratory phase) of the patient based on the sensor signal from sensor 12A. In some examples, a midpoint of each respiratory phase is determined based on zero crossings of the sensor signal. A zero crossing refers to a portion of a respiratory waveform (obtained via a filtered accelerometer signal) at which an amplitude changes from (e.g., crosses over) a negative amplitude to a positive amplitude (or vice versa) with the amplitude having a value of zero at the crossing point. In some examples, the midpoint may sometimes be referred to as a midpoint of a slope of an inspiratory phase or a midpoint of a slope of an expiratory phase of the respiratory waveform. The extracted features (e.g., midpoints) may be used by control portion 14A to control (e.g., synchronize, trigger, etc.) the delivery of therapy relative to the appropriate respiratory phase of the patient, for diagnostic storage and/or display, and/or for other purposes.

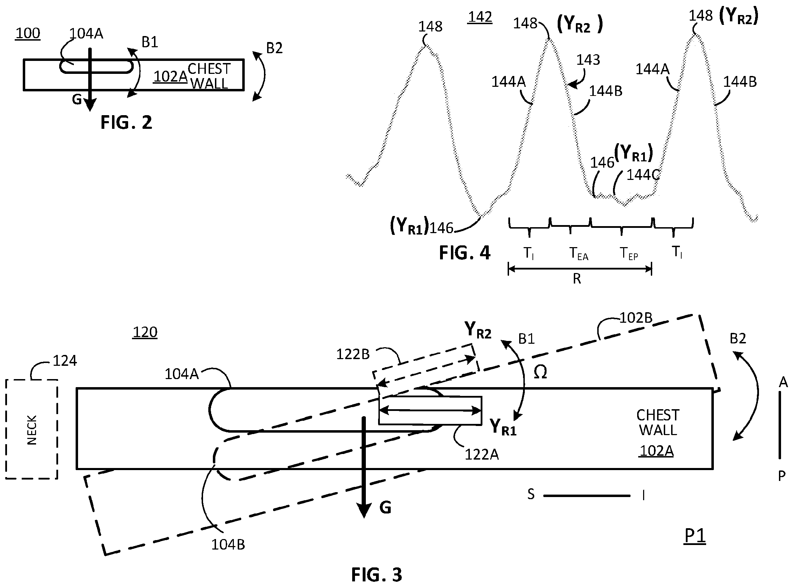

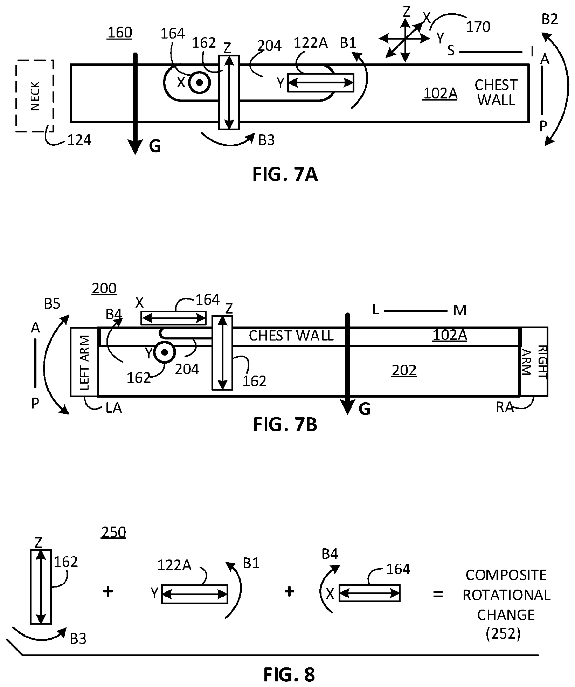

In one example, sensor 12A may include a single axis accelerometer having one measurement access to provide one corresponding sensor signal as will be described below with reference to at least FIG. 2. In other examples, sensor 12A may include a multiple axis accelerometer having more than one measurement axis, such as at least two orthogonal measurement axes to provide two corresponding sensor signals or three axes (e.g., a three axis accelerometer) to provide three corresponding sensors signals as will be described below with reference to at least FIGS. 7A-8. Each sensor signal may be filtered by linear filters (e.g., lowpass, highpass, bandpass) or non-linear filters (e.g., median filter) to recover the low-frequency respiration signal while rejecting measurement noise, muscle noise, cardiac noise, and/or other noise.

In some examples, at least some of the example methods (and/or example devices) of the present disclosure regarding respiratory determinations may be performed (or comprise) a single three axis accelerometer sensor without (i.e., while excluding) a second sensing element. In some examples, the single three axis accelerometer is an implantable element. However, it will be understood that at least some examples (e.g., methods and/or devices) of the present disclosure may comprise at least one sensing element in addition to a single three axis accelerometer. In some such examples, the second sensing element may be external to the patient's body while in some examples, the second sensing element may be implanted within the patient's body. In some of these examples, the implantable second sensing element may be located within (or on) the same device as the single three axis accelerometer or may be located as or in/on a separate implantable element. The second sensing element may comprise an accelerometer, a magnetometer, a gyroscope, a sound sensor, and/or other types of sensors. In some such examples, the second sensing element (e.g., second accelerometer, other) may be located in a body region (e.g., abdomen, spine) different from a body region (e.g., thoracic, chest) in which the single three axis accelerometer is located.

FIG. 1B is a block diagram illustrating one example of a device 10B for detecting respiration and applying stimulation based on the detected respiration. Device 10B includes a sensor 12B and a control portion 14B. Sensor 12B may comprise one example implementation of sensor 12A (FIG. 1A) and/or control portion 14B may comprise one example implementation of control portion 14A (FIG. 1A). In this example, sensor 12B senses changes in acceleration indicative of respiration of the patient as indicated at 16.

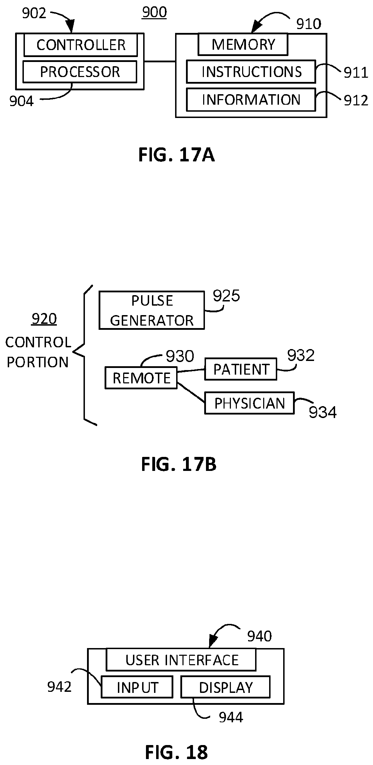

Control portion 14A (FIG. 1A) and/or control portion 14B (FIG. 1B) may comprise one example implementation of control portion 900 in FIG. 17A. Control portion 14B includes portions 18-28 to implement functions based on the sensor signal. At portion 18, control portion 14B may extract (e.g., identify) a feature or features of each respiratory phase based on the sensor signal(s) (e.g., as illustrated and described below with reference to FIGS. 4-5I). If sensor 12B provides more than one signal, features may be extracted from each sensor signal independently. In one example, the extracted features from one sensor signal may be selected for identifying each respiratory phase. The sensor signal may be selected based on a confidence factor to be described below. In other examples, the extracted features from each sensor signal may be combined to provide a composite signal (e.g., average of features, median of features, etc.) as will be described below with reference to at least FIG. 8.

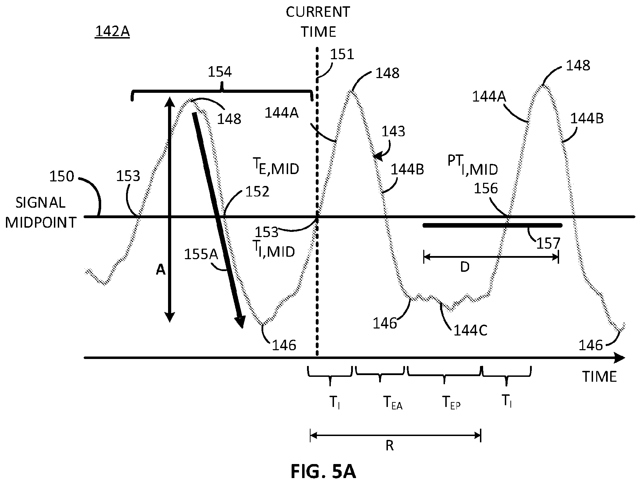

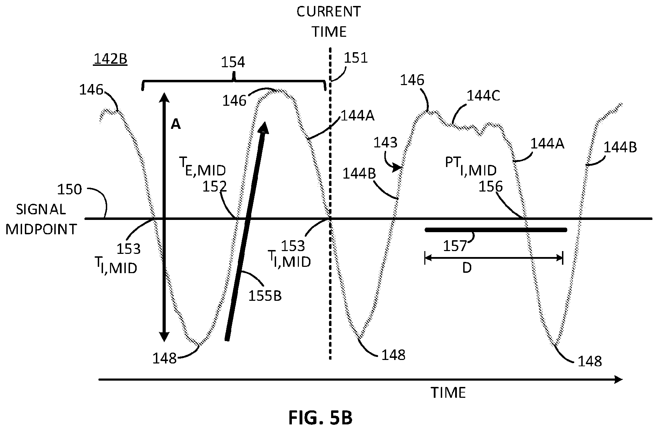

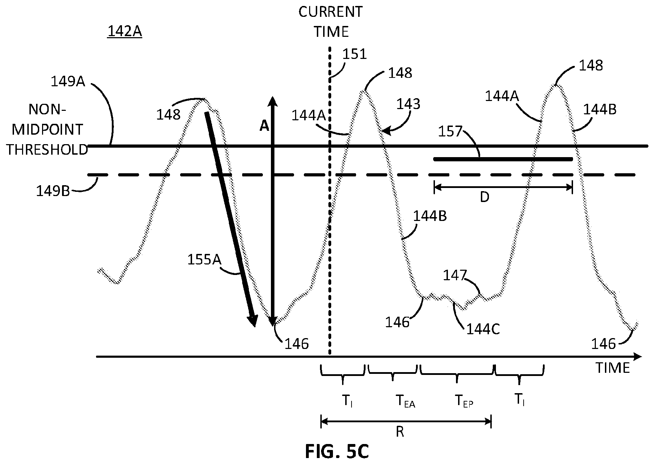

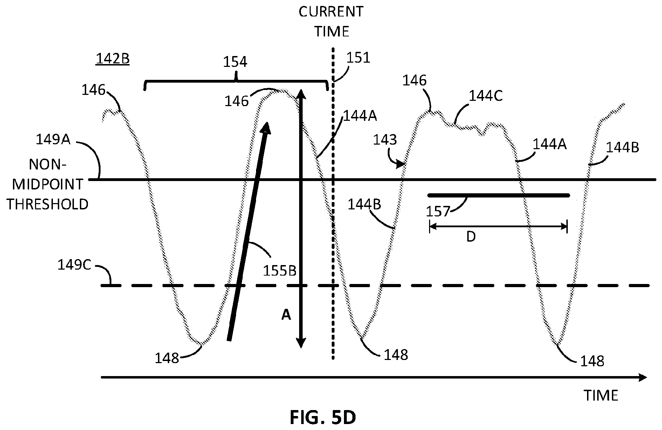

In one example, the extracted features include a midpoint of each respiratory phase of the patient, such as the midpoint 153 of each inspiratory phase and/or the midpoint 152 of each expiratory phase as will be described below in more detail at least with reference to FIGS. 5A and 5B. In some examples, the extracted features include the curvature of each peak (e.g., 148 of FIG. 4) versus each trough (e.g., 146 of FIG. 4) of the sensor signal(s). In other examples, the extracted features include relative peak-to-trough timing (e.g., from 146 to 148 and from 148 to 146 of FIG. 4) and/or peak-to-trough slope of the sensor signal(s). In yet other examples, the extracted features include the relative slope of the sensor signal(s) between peak and trough (e.g., from 148 to 146 of FIG. 4) versus between trough and peak (e.g., 146 to 148 of FIG. 4). In yet other examples, the extracted features may include inflection points and/or curvature (e.g., 147 of FIGS. 5C and 5D) of non-peak/non-trough locations (e.g., at the midpoint) of the sensor signal(s). In yet other examples, the extracted features include a non-midpoint threshold crossing of each inspiratory phase and/or a non-midpoint threshold crossing of each expiratory phase of the sensor signal(s) (e.g., 149 of FIGS. 5C and 5D).

At portion 20, control portion 14B may identify each respiratory phase of the patient based on the sensor signal(s). In some examples, the control portion 14B identifies each expiratory phase and/or each inspiratory phase of the patient based on the sensor signal(s), such as based on the features extracted from the sensor signal(s) by portion 18. In one example, the midpoints (e.g., zero crossings of a highpass filtered or bandpass filtered sensor signal) of each respiratory phase may be used to identify each inspiratory phase and/or each expiratory phase since the time between the midpoint of an expiratory phase followed by the midpoint of an inspiratory phase is typically longer than the time between the midpoint of an inspiratory phase followed by the midpoint of an expiratory phase (e.g., due to an expiratory pause of each expiratory phase).

In other examples, the feature of curvature of peak versus trough may be used to identify each inspiratory phase and/or each expiratory phase since the peak at an inspiratory phase to expiratory phase transition (e.g., 148 of FIG. 4) will typically have a sharper peak than the peak at an expiratory phase to inspiratory phase transition (e.g., 146 of FIG. 4). That is, the transition from inhalation to exhalation is typically much quicker than the transition from exhalation to inhalation. In yet other examples, the feature of relative peak-to-trough timing and/or peak-to-trough slope may be used to identify each inspiratory phase and/or each expiratory phase since the inspiratory phase is typically shorter than the expiratory phase, such that relative phase may be determined. In yet other examples, the features of relative slope of the sensor signal(s) between peak and trough versus between trough and peak may be used to identify each inspiratory phase and/or each expiratory phase since the slope of the expiratory phase (e.g., 144B of FIG. 4) is typically smaller than the slope of the inspiratory phase (e.g., 144A of FIG. 4). In yet other examples, the feature of inflection points and/or curvature of non-peak/non-trough locations (e.g., 147 of FIGS. 5C and 5D) may be used to identify each inspiratory phase and/or each expiratory phase since the expiratory phase may have extra inflection point(s) versus the inspiratory phase due to the longer time spent in the expiratory phase (e.g., due to an expiratory pause (e.g., 144C of FIG. 4) prior to the inspiratory phase). In yet other examples, the feature of a non-midpoint threshold crossing (e.g., 149 of FIGS. 5C and 5D) may be used to identify each inspiratory phase and/or each expiratory phase since the time between the non-midpoint threshold crossing of an expiratory phase followed by the non-midpoint threshold crossing of an inspiratory phase is typically longer than the time between the non-midpoint threshold crossing of an inspiratory phase followed by the non-midpoint threshold crossing of an expiratory phase (e.g., due to an expiratory pause of each expiratory phase).

Accordingly, examples of the present disclosure may use any one of the above-described extracted features (e.g., midpoint, peak-to-trough timing or slope, relative slope, inflection points of non-peak or non-trough locations, etc.) to recognize differences in the respiratory waveform which may be used to reliably and uniquely identify an inspiratory phase and an expiratory phase (and vice versa) within a respiratory waveform.

With this in mind, in some examples the identification of the respective inspiratory and expiratory phases (e.g., via control portion 20) by looking at phase differences based on an extracted feature may sometimes be referred to as an example method of identifying feature-based phase differences (i.e., feature-based phase difference identification).

At least some example methods may use such identification of feature-based phase differences to determine a respiratory phase, a respiratory rate, and durations of inspiration/expiration, among other useful respiratory parameters, therapy parameters, etc.

For greater context, it may be understood that respiratory signals generally include two opposite phases: positive and negative (e.g., in/out, inhalation/exhalation) so the signal captured by a sensor may capture these two different polarities of the signal. Like many biological signals, a magnitude of each polarity/phase may vary due to many factors, such as sleep stage, patient phenotype (e.g., body type), and a myriad of other factors. Thus, the absolute limits of the magnitude of the signal may be very different from patient to patient and from moment to moment.

However, in at least some example methods of the present disclosure, identifying featured-based phase differences provides for an adaptive method that accounts for these ongoing variations in signal magnitude such that the example method will be robust even when a patient changes posture, sleep angle (e.g., due to bed, chair, pillow, etc.), sleep stage, different body types, etc. The example methods also may be well suited to handle severe changes in signal morphology such as inversion, where due to changes in body posture, the sensor signal polarity associated with inspiration (i.e., inhalation) and expiration (e.g., exhalation) may become reversed. In such situations, the example methods which look at feature-related differences between the phases are well suited to handle inversion because such methods looks at differences between the phases that are unrelated to the polarity of the signal.

Among other aspects, at least some of the example methods of respiratory determination for use with and/or in implantable medical devices may be highly efficient, which eliminates unnecessary power use while also providing computation time for other significant tasks of the implantable medical device. Efficiency is particularly relevant when processing multiple sensor signals simultaneously, such as three signals from a three axis accelerometer. With this in mind, at least some example methods which use feature-based phase difference identification (e.g., identifying midpoints, slopes, relative slopes, etc.) are computationally simple and efficient to be performed simultaneously on multiple sensor signals.

Other respiratory determination techniques require time to converge where the techniques operate over several breaths (e.g., 3 to 30 breaths depending on the design and initial values) prior to extracting features from the sensor signal that are trustworthy. In addition, some of these techniques may depend on knowing the average signal value, root mean square (RMS) value, or an historical rate to work properly. In sharp contrast, at least some example methods of respiratory phase determination of the present disclosure which use feature-related phase difference identification (e.g., comparison of midpoints, slopes, timing, etc.) can be used immediately on the first breath without any loss in accuracy.

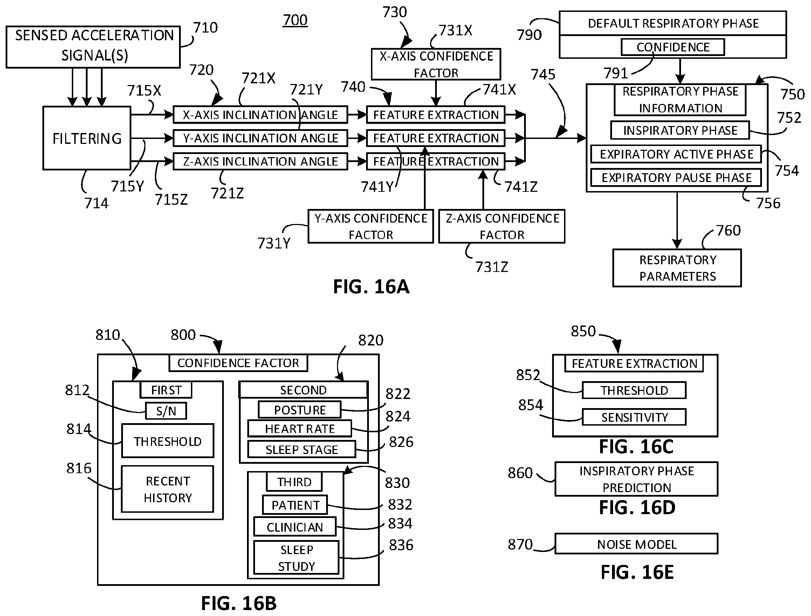

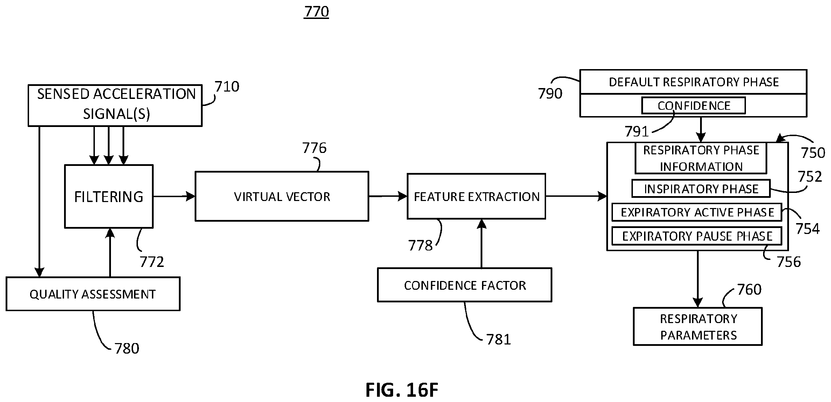

With further reference to FIG. 1B, at portion 22 control portion 14B may determine a confidence factor for each respiratory phase. Control portion 14B may determine the confidence factor associated with each respective identified expiratory phase and/or identified inspiratory phase from portion 20. The confidence factor is an estimated probability of correctness of the phase determination. The confidence factor may be determined for each extracted feature and/or each signal/axis as described in more detail below and with reference to at least FIGS. 16A, 16B, and 16F.

In one example, control portion 14B may determine the confidence factor associated with each respective identified expiratory phase and/or identified inspiratory phase from portion 20 using a statistical test. The statistical test may be used for an inspiration/expiration timing ratio not equal to 1.0 (i.e., relative duration is not 1:1) over one or more historical median calculations using the historical median calculation data points as the sample population for a student's T-test (or other statistical test) for a null hypothesis of a mean of 0 difference between inspiration and expiration time (or inspiration/expiration midpoint timing). This statistical test uses both the sign and magnitude information from the median calculations to inform the confidence level (e.g., noisy signal centered on 0 would have low confidence, a clean signal that is either strongly positive or strongly negative would have high confidence). The t-test (or other statistical test) may be based on a lookup table and may optionally include a saturation or extrapolation at high and/or low confidence levels. In other examples, the confidence factor may be partly or fully determined based on a signal-to-noise ratio, relative signal amplitude between axes, and/or absolute signal amplitude. In some examples, the confidence factors may be programmatically biased (e.g., by design or by a clinician) for each axis, such as to modify and/or override other confidence factors.

In other examples, confidence factors may be determined based on an expected number and/or timing of zero crossings (e.g., used to extract midpoints of respiratory phases). In one example, an expected number of zero crossings may be based on a running respiration rate, such as the expected number of zero crossings in an estimated respiration period. In another example, an expected number of zero crossings may be fixed by a physiologic interval (e.g., expect no more than 3 zero crossings per a physiologically realistic interval). In other examples, confidence factors may be determined based on the expected timing from one zero crossing to the next zero crossing. In some examples, a violation of the expected number of zero crossings and/or the timing of zero crossings may be termed an "excess zero" and may be rejected on that basis. In other examples, excess zeros may be used to inform the confidence factor (e.g., more excess zeros equals lower signal quality) or may itself be used as a confidence factor. More excess zeros may occur in one respiratory phase than in the other respiratory phase (e.g., an expiratory phase may have more excess zeros than an inspiratory phase), which may be used to inform the confidence in the current polarity determination described below with reference to at least FIGS. 3-5B.

In some examples, the control portion 14B may compute a confidence factor as a statistical test for a median of the sensor signal(s) (e.g., data points of the sensor signals(s)) being different than a mean of the sensor signal(s). In other examples, the control portion 14B may compute a confidence factor based on a magnitude of an AC component of the sensor signal(s). In other examples, the control portion 14B may compute a confidence factor as a magnitude of an AC component of the sensor signal(s) divided by a root mean square of the sensor signal(s). In other examples, the control portion 14B may compute a confidence factor as a magnitude of a first harmonic of the sensor signal(s) divided by a sum of one or more (e.g., at least one) magnitudes of higher order harmonics of the sensor signal(s). In other examples, the control portion 14B may compute a confidence factor as a ratio of an AC component of the sensor signal(s) to a noise floor of the sensor 12B. In yet other examples, the control portion 14B may compute a confidence factor as a linear or non-linear combination of any of the above example confidence factors.

In some examples, the control portion 14B determines a confidence factor associated with each of three respective sensor signals (e.g., from a three axis accelerometer), identifies which respective sensor signal exhibits the greatest confidence factor, and determines the midpoint (or other features) of each respiratory phase of the patient based on the identified sensor signal exhibiting the greatest confidence factor. In other examples, control portion 14B may determine a confidence factor associated with each of the three respective sensor signals over a moving period (i.e., rolling window), identify which respective sensor signal exhibits the highest mean or median confidence over the moving period, and determine the midpoint (or other features) of each respiratory phase of the patient based on the identified sensor signal exhibiting the highest mean or median confidence over the moving period. In some examples, once a sensor signal is identified for determining the midpoint (or other features) of each respiratory phase, the identified sensor signal may be used to determine the midpoint (or other features) of each respiratory phase for at least a predetermined period or for a predetermined number of respiratory cycles. In this case, once the predetermined period has elapsed or the predetermined number of respiratory cycles has been reached, control portion 14B may identify another sensor signal (as described above) for determining the midpoint (or other features) of each respiratory phase or may determine that continued use of the same sensor signal remains preferable. In yet other examples, control portion 14B may intentionally alternate between sensor signals having a confidence factor above a predefined threshold for determining the midpoint (or other features) of each respiratory phase. In yet other examples, control portion 14B may select two out of the three sensor signals for determining the midpoint (or other features) of each respiratory phase in response to the sensor signals matching in inspiration timing (e.g., start and end of inhalation) within a predetermined or dynamic margin.

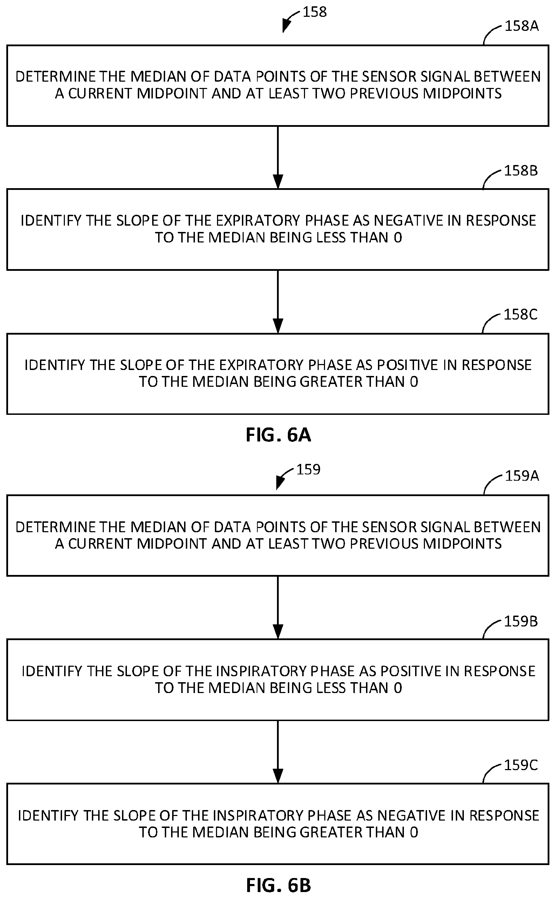

At portion 24, control portion 14B may identify a slope of each respiratory phase. In one example, control portion 14B may identify whether the slope of each expiratory phase is positive or negative. One example of identifying whether the slope of each expiratory phase is positive or negative is based on the extracted midpoints and will be described below at least with reference to FIG. 6A. In other examples, control portion 14B may identify whether the slope of each inspiratory phase is positive or negative. One example of identifying whether the slope of each inspiratory phase is positive or negative is based on the extracted midpoints and will be described below at least with reference to FIG. 6B. In some examples, the slope of each expiratory phase may be identified as positive or negative without determining a value of the slope of each expiratory phase. Likewise, the slope of each inspiratory phase may be identified as positive or negative without determining a value of the slope of each inspiratory phase.

In other examples, the feature of a non-midpoint threshold for each inspiratory phase and/or each expiratory phase may be used to identify whether the slope of each inspiratory phase and/or each expiratory phase is positive or negative. The slope may be determined by computing the mean and median of the sensor signal over a full cycle window and comparing the mean to the median. If the mean is greater than the median, then the direction of the slope of the expiratory phase is negative (and thus the slope of the inspiratory phase is positive). If the mean is less than the median, then the slope of the expiratory phase is positive (and thus the slope of the inspiratory phase is negative).

At portion 26, control portion 14B may predict a future inspiratory phase. In one example, control portion 14B predicts a midpoint of a future inspiratory phase based on previous midpoints of each respiratory phase. In some examples, the midpoint of a future inspiratory phase may be predicted based on one or more previous respiratory cycles and the respiratory rate of one or more previous respiratory cycles. For example, the midpoint of a future inspiratory phase may be predicted based on the mean of previous midpoints, the median of previous midpoints, a linear extrapolation of previous midpoints, or a non-linear extrapolation of previous midpoints. Previous values, which are advantageous as "look back" techniques, may be used to improve accuracy of feature extraction and to reduce susceptibility to a noisy signal (i.e., non-respiratory transients) due to limb movement, bed partner movement, etc. For example, zero crossing detection (e.g., as one example of midpoint detection for a highpass or bandpass filtered signal) accuracy may be improved by using data points before and after the zero crossing. In other examples, the midpoint of a future inspiratory phase may be predicted based on similarity (e.g., cross-correlation) with a reference respiratory morphology.

With the midpoint of the future inspiratory phase predicted, the beginning of the future inspiratory phase may be predicted by subtracting a first predetermined interval (e.g., a first time period measured in seconds and/or milliseconds) from the predicted midpoint and the end of the future inspiratory phase may be predicted by adding a second predetermined interval (e.g., a second time period measured in seconds and/or milliseconds) to the predicted midpoint. In other examples, the future inspiratory phase may be predicted based on other previously extracted features, such as previous curvatures of peak versus trough, previous relative peak-to-trough timing and/or peak-to-trough slope, previous inflection points and/or curvature of non-peak/non-trough locations, and/or previous non-midpoint thresholds as previously described.

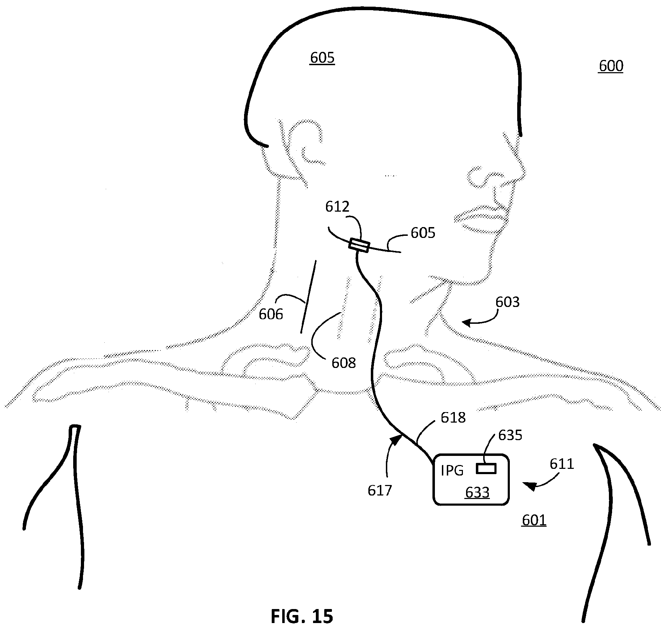

At portion 28, control portion 14B may apply stimulation. In one example, device 10B may include an electrode (e.g., electrode 612 to be described with reference to at least FIG. 15) to deliver electrical stimulation to an upper airway patency-related nerve of the patient or another nerve of the patient. In one example, the control portion 14B may apply the electrical stimulation based on the predicted midpoint of the future inspiratory phase. The control portion 14B may apply the electrical stimulation with a time or ratio-based offset from the predicted midpoint of the future inspiratory phase. For example, the control portion 14B may apply the electrical stimulation starting relative to the predicted midpoint by a first predetermined interval (e.g., first time period) and ending after the predicted midpoint by a second predetermined interval (e.g., second time period). The first predetermined interval may be selected based on a preselected, or computed, relative position (e.g., 20% or 30%) within the mean or median of previous inspiratory phase durations plus optionally an absolute additional amount (e.g., 200 ms or 300 ms) to ensure the stimulation is started just prior to the inspiratory phase. The second predetermined interval may be selected based on a preselected, or computed, relative position within the mean or median of previous inspiratory phase durations plus optionally an absolute additional amount to ensure the stimulation is ended at or just after the inspiratory phase. The relative position may be responsive to optimize therapy based on detected sleep disordered breathing like obstructive sleep apnea, central sleep apnea, multiple-type sleep apneas, etc., and may be moved forwards or backwards from the relative position to optimize the efficacy of the stimulation. In other examples, control portion 14B may apply stimulation based on future inspiratory phases predicted based on other previously extracted features, such as previous curvatures of peak versus trough, previous relative peak-to-trough timing and/or peak-to-trough slope, previous inflection points and/or curvature of non-peak/non-trough locations, and/or previous non-midpoint threshold crossings as previously described. Additional examples of the application of stimulation will be described below with reference to FIGS. 5A-5D.

In some examples, the stimulation portion 28 controls stimulation of target tissues, such as but not limited to an upper airway patency nerve (e.g., hypoglossal nerve, ansa cervicalis-related nerve, other) and/or a phrenic nerve, to treat sleep disordered breathing (SDB) behavior. In some examples, the stimulation portion 28 comprises a closed loop parameter to deliver stimulation therapy in a closed loop manner such that the delivered stimulation is in response to and/or based on the sensor signal(s).

In some examples, the closed loop parameter may be implemented using the sensed information to control the particular timing of the stimulation according to respiratory information, in which the stimulation pulses are triggered by or synchronized with specific portions (e.g., inspiratory phase) of the patient's respiratory cycle(s). In some such examples and as previously described, this respiratory information may be determined via the sensor 12A or 12B.

In some examples in which the sensor signal enables determining at least respiratory phase information, the closed loop parameter may be implemented to initiate, maintain, pause, adjust, and/or terminate stimulation therapy based on (at least) the determined respiratory phase information. In some examples, the stimulation is started prior to an onset of the inspiratory phase and the stimulation is stopped exactly at the end of the inspiratory phase or stopped just after the end of the inspiratory phase.

In some examples the stimulation portion 28 comprises an open loop parameter by which stimulation therapy is applied without a feedback loop. In some such examples, in an open loop mode the stimulation therapy is applied during a treatment period without (e.g., independent of) information sensed regarding the patient's sleep quality, sleep state, respiratory phase, AHI, etc. In some such examples, in an open loop mode the stimulation therapy is applied during a treatment period without (i.e., independent of) particular knowledge of the patient's respiratory cycle information. However, in some such examples, some sensory feedback may be utilized to determine, in general, whether the patient should receive stimulation based on a severity of sleep apnea behavior.

In some examples the stimulation portion 28 comprises an auto-titration parameter by which an intensity of stimulation therapy can be automatically titrated (i.e., adjusted) to be more intense (e.g., higher amplitude, greater frequency, and/or greater pulse width) or to be less intense within a treatment period. In some such examples, such auto-titration may be implemented based on sleep quality, which may be obtained via sensed physiologic information, in some examples. It will be understood that such examples may be employed with synchronizing stimulation to sensed respiratory information (i.e., closed loop stimulation) or may be employed without synchronizing stimulation to sensed respiratory information (i.e., open loop stimulation). In some examples, at least some aspects of the auto-titration parameter may comprise, and/or may be implemented, via at least some of substantially the same features and attributes as described in Christopherson et al., US 8,938,299, SYSTEM FOR TREATING SLEEP DISORDERED BREATHING, issued January 20, 2015.

In some examples, the stimulation portion 28 of control portion 14B may comprise an "off period" function by which a user or clinician may adjust the time that stimulation will remain off and which may be expressed as a percentage of the previous "on period." In some examples, the "off period" for stimulation coincides with the expiratory active phase. However, in some examples, once enabled by the user or clinician, the "off period" (i.e., no stimulation) setting is implemented regardless of detected phases. In some examples, the "on period" may sometimes be referred to as a stimulation period and the "off period" may sometimes be referred to a non-stimulation period. In some examples, a stimulation cycle may comprise one stimulation period followed by one non-stimulation period. In some examples, the stimulation cycle may have a duration corresponding to a duration of a respiratory cycle.

In some examples, the stimulation portion 28 of control portion 14B may comprise a "maximum stimulation" function which may be used by a patient or clinician to adjust a maximum time for an "on period" of stimulation for a given stimulation cycle, after which an "off period" takes place. The "on period" may extend for a selectable, predetermined period of time. In some examples, the "on period" for stimulation coincides with the inspiratory phase. In some such examples, once enabled by the user or clinician, the "on period" of stimulation is implemented regardless of detected phases.

FIG. 2 is a diagram including a side view, schematically representing an example method 100 and/or example device including a sensor 104A. In one example, sensor 104A may provide sensor 12A or 12B of FIGS. 1A and 1B. As shown in FIG. 2, example sensor 104A is chronically, subcutaneously implanted within a chest wall 102A of a patient's body. During breathing, the chest wall 102A will exhibit rotational movement (B2) as at least some portions of the chest wall 102A move (e.g., rise and fall) during inspiration and expiration, wherein inspiration corresponds to expansion of the rib cage and expiration corresponds to contraction of the rib cage. During this expansion and contraction of the rib cage during breathing, at least some portions of the chest wall 102A exhibit rotational behavior, which may in turn may be sensed upon the sensor 104A experiencing such rotational movement (as represented via directional arrow B1), which in turn provides respiration information as described above and as further described below. It will be understood, as further described later, that the rotational movement of the sensed portion of the chest wall 102A is not necessarily or strictly limited to rotational movement in a single plane. In some examples, sensor 104A may comprise a portion of a larger device, as further described later in association with at least FIG. 15.

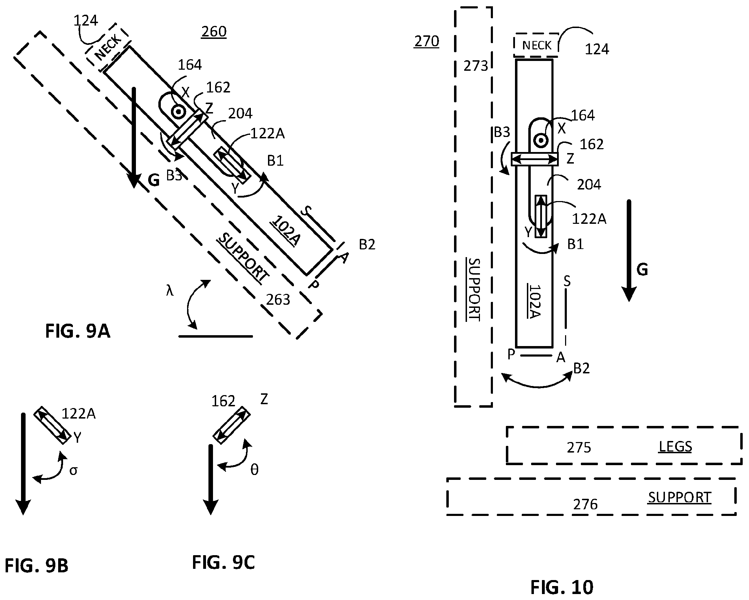

As further shown in FIG. 2, the sensor 104A may sense the rotational movement of at least a portion of the chest wall 102 (as represented via directional arrow B2) relative to an earth gravitational field (arrow G), i.e., gravity vector. It is noted that the sensor 104A may predominately sense rotation, but may also sense some linear acceleration component. For illustrative simplicity to depict at least some examples, FIG. 2 depicts the chest wall 102A as if the patient's body was in a generally horizontal sleep position. It will be understood that at least some example devices and/or example methods will be effective in detecting respiration information regardless of whether the generally horizontal sleep position is a supine position, a prone position, or a side-laying (i.e., lateral decubitus) position. Moreover, at least some example methods and/or example devices also will be effective in detecting respiration information if, and when, the patient is in positions other than a generally horizontal position, such as sitting in a chair in a vertically upright position, in a reclining position, etc. as further described later in association with at least FIGS. 9A-10.

Moreover, determining respiration information via acceleration-based sensing of rotational movements (at a portion of a chest wall of the patient) does not include, or depend on, determining (e.g., via sensing) a body position of the patient. Accordingly, while such respiration information may be determined in any one of several different sleeping body positions, such determination may be performed without determining the particular sleeping body position at the time the sensing of the rotational movements is being performed.

However, in some such examples, the particular sleeping body position occurring at the time of the determining the respiration information (via acceleration-based sensing of rotational movements of a portion of a chest wall during breathing) may be determined and used as a parameter to augment the determined respiration information and/or other general patient physiologic information, in some instances.

In some examples, securing the implantable acceleration sensor(s) comprises mechanically coupling the sensor(s) relative to a respiratory body portion. In some examples, securing the implanted acceleration sensor(s) comprises securing the acceleration sensor relative to tissue on top of a muscle layer of the respiratory body portion, while in some examples the sensor may be secured directly to a muscle layer of the respiratory body portion. In some examples, the acceleration sensor may be secured subcutaneously within the respiratory body portion without securing the acceleration sensor on the muscle layers. In some examples, the respiratory body portion may comprise the chest. In some such examples, the respiratory body portion may comprise a portion of the chest, such as but not limited to a portion of a chest wall. In some instances, the portion of the chest wall may correspond to a portion of the rib cage. In some examples, such aspects of securing the sensor(s) relative to a muscle layer or subcutaneously are also applicable to securing the sensor at other respiratory body portions, such as an abdomen (e.g., abdominal wall) by physically (e.g., mechanically) coupling the sensor relative to the abdomen to sense rotational movement at the abdomen during breathing.

FIG. 3 is a diagram 120, including a side view, schematically representing an example method and/or example sensor 104A. As shown in FIG. 3, in some examples the sensor 104A may comprise a sensing element 122A, which is arranged to measure an inclination angle ($\Omega$) upon rotational movement of the sensing element 122A caused by breathing. As represented in FIG. 3, upon rotational movement of at least a portion of the chest wall 102A during breathing, the sensing element 122A may rotationally move between a first angular orientation YR1 (shown in solid lines) and a second angular orientation YR2 (shown in dashed lines). In some such examples, the first angular orientation YR1 (shown in solid lines) of sensing element 122A may correspond to a peak expiration of a respiratory cycle (e.g., rib cage contracted) and the second angular orientation YR2 (shown in dashed lines) of sensing element 122A may correspond to a peak inspiration of the respiratory cycle (e.g., rib cage expanded).

With reference to at least FIG. 3, it will be understood that the sensing element 122A moves with at least a portion of the chest wall 102A as depicted in dashed lines. Accordingly, sensing element 122A does not move relative to the chest wall 102A, but rather the sensing element 122A rotationally moves along with (e.g., in synchrony with) the rotational movement of at least the portion of the chest wall 102A (in which the sensor 104A, including sensing element 122A), is implanted) during breathing.

In some examples, the sensing element 122A comprises an accelerometer, which may comprise a single axis accelerometer in some examples or which may comprise a multiple-axis accelerometer in some examples. Via the accelerometer, the sensing element 122A can determine absolute rotation of sensor 104A (and therefore rotation of the portion of the chest wall 102A) with respect to gravity (e.g., earth gravity vector G), rather than instantaneous changes in rotation. In some examples, element 122A may comprise a single axis accelerometer to measure (at least) a value of, and changes in the value of, the above-noted inclination angle ($\Omega$) associated with movement of at least a portion the chest wall 102 caused by breathing.

FIG. 4 is a diagram including a graph schematically representing a filtered acceleration signal 142 sensed via a sensor, such as sensing element 122A in FIG. 3. In some examples described herein, filtered acceleration signal 142 may be referred to as a breathing signal $b(t)$. As shown in FIG. 4, signal 142 corresponds to a respiratory waveform exhibited through several respiratory cycles during breathing. Each respiratory cycle 143 comprises an inspiratory phase (Ti) 144A, an expiratory active phase ($T_{EA}$) 144B, and an expiratory pause phase ($T_{EP}$) 144C. An expiratory phase includes both the expiratory active phase and the expiratory pause phase. It will be understood that the example respiratory waveform in FIG. 4 represents a typical respiratory waveform for at least some patients during normal breathing, but not necessarily for all patients at all times.

With FIG. 4 in mind, and with further reference to FIG. 3, it will be noted that the first angular orientation YR1 of sensing element 122A (shown in solid lines) may correspond generally to a peak expiration 146 (e.g., end of the expiratory active phase ($T_{EA}$)) while the second angular orientation YR2 of sensing element 122A (shown in dashed lines) may correspond to a peak 148 of inspiratory phase (Ti), i.e., the end of inspiration just at or before the onset of expiration.

With further reference to FIG. 3, upon rotation (B2) of at least a first portion of chest wall 102A, as represented by directional arrow B2 and the depiction of chest wall in dashed lines 102B, such as during inspiration, the sensing element 122A rotates by the inclination angle ($\Omega$) with chest wall 102A to a position or orientation YR2 shown in dashed lines 122B. Upon the end of inspiration, and the ensuing expiration, the chest wall 102 will rotate back into the position shown in solid lines (e.g., end of expiration) such that the sensing element 122A will sense a change in inclination angle ($\Omega$) from the position YR2 (shown in dashed lines) back to the position YR1 (shown in solid lines).

In sensing the inclination angle ($\Omega$) through successive respiratory cycles, the sensing element 122A obtains an entire respiratory waveform, which may comprise information such as the duration, magnitude, etc. of an inspiratory phase (Ti), expiratory active phase ($T_{EA}$), and expiratory pause phase ($T_{EP}$) of respiratory cycles of the patient, and/or other information (e.g., respiratory rate, etc.) as represented in FIG. 4 and/or as further described later. With this in mind, in some examples the obtained respiratory waveform (e.g., respiration morphology) also comprises a respiratory period, which includes the inspiratory phase, the expiratory active phase, and the expiratory pause phase. In one aspect, the respiratory period corresponds to a duration (R) of a respiratory cycle, with this duration comprising a sum of a duration of the inspiratory phase, a duration of the expiratory active phase, and a duration of the expiratory pause phase.

In some examples, the identified respiration morphology comprises identifying (within the respiratory waveform morphology) a start of the inspiratory phase, i.e., onset of inspiration. In some examples, this start of the inspiratory phase also may at least partially correspond to an expiration-to-inspiration transition. In some examples, a method of identifying the start of the inspiratory phase within the identified respiratory waveform morphology further comprises performing the identification (of the start of the inspiratory phase) without identifying an end (e.g., offset) of the inspiratory phase, which may enhance the accuracy of identification (of the start of the inspiratory phase) in the presence of noise, in contrast to identification of more than one phase transition (e.g., inspiratory-to-expiratory or expiratory-to-inspiratory) per respiratory cycle where each transition is subject to mis-identification due to noise. In some such examples, the end (e.g., offset) of the inspiratory phase corresponds to a start (e.g., onset) of the expiratory active phase.

In some examples, at least some aspects of such identification, prediction, etc. of features (e.g., start of inspiratory phase, end of expiration, etc.) within a respiratory waveform may be implemented via at least some of substantially the same features and attributes as later described in association with at least FIGS. 16A-16E and/or various examples throughout the present disclosure, such as but not limited to identifying inspiratory phase (e.g., 752 in FIG. 16A), inspiratory phase prediction (e.g., 860 in FIG. 16D), etc.

With further reference to FIG. 3, it will be understood that the second angular orientation YR2 of sensing element 122A is not a fixed position, but rather corresponds to a temporary position at one end (e.g., a second end) of a range of rotational movement of the portion of the chest wall 102A, such as peak inspiration 148 (FIG. 4) during breathing. This second end of the range of rotational movement (and therefore the second angular orientation YR2) may vary depending upon whether the patient is exhibiting normal/relaxed breathing, forced breathing (such as more forceful inspiration), and/or disordered breathing. Moreover, this second end of the range of rotational movement may exhibit some variance from breath-to-breath even during relaxed breathing.

Similarly, the first angular orientation YR1 of sensing element 122A shown in FIG. 3 does not comprise a fixed position, but rather the first angular orientation YR1 corresponds to a temporary position at an opposite other end (e.g., a first end) of a range of rotational movement of the portion of the chest wall 102A, such as peak expiration 146 (FIG. 4) during breathing. This first end of range of rotational movement (and therefore the first angular orientation YR1) may vary depending upon whether the patient is exhibiting normal/relaxed breathing, forced breathing (such as more forceful expiration), and/or disordered breathing. Moreover, this first end of the range of rotational movement may exhibit some variance from breath-to-breath even during normal/relaxed breathing.

While there may be some variances in the exact rotational positions of the respective second angular orientation YR2 and/or the first angular orientation YR1, it will be understood that there consistently will be a significant difference (i.e., substantial difference) between the first angular orientation YR1 and the second angular orientation YR2, by which respiration morphology (e.g., shown in FIG. 4) can be determined as the sensor moves through the full range of rotational movement between the first and second angular orientations, YR1 and YR2 during patient breathing. Moreover, the variances in the particular rotational position of the first angular orientation YR1, and of the second angular orientation YR2, at the ends of the range of rotational movement of the sensing element 122A may yield valuable information regarding variances in respiration, such as variances in amplitude of inspiration and/or expiration, variances in respiratory rate, etc. In some examples, the ends of the range of angular movement between the two orientations YR1, YR2 may correspond to the ends of a range of values of an AC signal component of the acceleration signal from the sensor.

In some examples, the first angular orientation YR1 may sometimes be referred to as a reference angular orientation, at least to the extent that the first angular orientation YR1 may correspond to an orientation which is the closest to being generally perpendicular to the gravity vector G for at least some sleeping body positions, such as but not limited to a generally horizontal sleep position.

With further reference to FIG. 3, in this example implementation and in general terms, the sensor 104A may be implanted in a manner to cause the first angular orientation YR1 (i.e., base orientation) of the measurement axis of the sensing element 122A to be generally parallel to a superior-inferior (S-I) orientation of at least the chest region of the patient's body, and generally perpendicular to an earth gravitational field G, such as when the patient is in a generally horizontal position. In this example implementation, the measurement axis of the sensing element 122A also may be understood as having an orientation generally perpendicular to an anterior-posterior (A-P) orientation of at least a portion of the chest region 102A of the patient's body.

In the example of FIG. 3, rotational movement of sensing element 122A, which has a Y-axis orientation, occurs roughly near or within a plane P1 defined by the anterior-posterior orientation (A-P) and by the superior-inferior orientation (S-I) of the patient's body. This rotational movement is primarily indicative of rotational movement of the rib cage during breathing, such as during a treatment period in which a patient is sleeping. Additional examples later describe additional/other aspects in which rotational movements of the rib cage are further indicative of breathing, and therefore respiratory morphology.

It will be understood that, due to patient-to-patient variances in anatomy and/or due to the particular location where the sensor 104A is actually implanted along the chest wall 102A, the sensing element 122A may extend in an orientation which is not exactly parallel to a superior-inferior orientation the chest wall (or entire patient as a whole), and not exactly perpendicular to an anterior-posterior orientation of the patient's body (and gravity vector G when laying in a generally horizontal position).

Nevertheless, in some example implementations, by arranging the measurement axis (Y) of the sensing element 122A to have an orientation as close as possible to being generally perpendicular to the gravity vector (G) for at least some patient body positions (e.g., generally horizontal sleep position), the sensitivity of the AC signal component of the acceleration sensing element 122A is maximized (and absolute value of the DC signal component is minimized), which in turn may increase the effectiveness of measuring changes in the inclination angle ($\Omega$) of sensing element 122A caused by, and during, breathing by the patient. In one aspect, the AC signal component of the acceleration sensing element 122A may be understood as the time-varying portion of the output signal of the acceleration sensing element 122A.

In particular, by arranging the sensing element 122A within the chest wall 102A to be as close as reasonably practical to being generally perpendicular to the earth gravitational field G (at least when the patient is in a primary sleep position), the sensed inclination angle will correspond to a maximum value of a measured AC component of the acceleration signal and a minimum absolute value of a measured DC component of the acceleration signal. Stated differently, when a measurement axis of the acceleration sensing element 122A is generally perpendicular (or as close as reasonably practical) to an orientation in which it would otherwise measure a maximum value (e.g., 1 g, such as when parallel to an earth gravity vector), the absolute value of the DC component will be negligible or minimal. In this situation, changes in value of the AC component of the acceleration signal become more prominent, being of a magnitude and/or reflecting significantly measurable changes as an orientation of the (measurement axis of the) acceleration sensing element changes (e.g., inclination angle) as the portion of the chest wall exhibits rotational movement caused by breathing.

It will be understood that based on the particular orientation at which the sensor 104A (e.g., sensing element 122A) is actually implanted, based on the varying position of the patient once the sensor 104A has been implanted, and/or based on other factors described further below, the measurement axis of the sensing element 122A at the chest wall 102A may not be perpendicular to the earth gravitational field G at the time of performing the sensing during breathing and hence the sensitivity of the AC component of the acceleration signal may not be at a maximum value. Nevertheless, at least some example methods (and/or devices) may perform the sensing (e.g., of the inclination angle of the sensing element 122A) to obtain the desired respiration information provided that the sensed signal provides a sufficiently high degree of sensitivity of a measured AC component of the acceleration signal. In some such examples, the methods and/or devices may employ magnitude criteria by which it may be determined if, and/or when, a sufficiently high degree of sensitivity of the measured AC signal is present. For instance, in some non-limiting examples, a sufficiently high degree of sensitivity corresponds to a measured AC signal having adequate signal-to-noise ratio in order to determine respiration.

The DC component of the acceleration signal may be determined using a low-pass filter (e.g., IIR, FIR, other low-pass type), a moving average (e.g., mean, median, other average type), or another suitable process. The AC component of the acceleration signal may be determined using a high-pass filter (e.g., IIR, FIR, other high-pass type), by subtracting the moving average from the signal average (mean, median, other average type), or another suitable process. In some examples, the filter band pass may be selected to emphasize the respiratory signal while rejecting non-respiratory signals. In some such examples, an output acceleration signal of sensing element 122A corresponds to a sine of the angle between the accelerometer measurement axis (i.e., orientation of Y) and a generally horizontal orientation (which is generally perpendicular to gravity vector G).

Because of variances in patient-to-patient anatomy, in some example methods/devices, an absolute magnitude of the AC signal component is not used to determine respiration information. Rather, by using the difference in magnitude of the value of the AC signal component between the first angular orientation (YR1) and the second angular orientation (YR2), the example methods/devices can determine a respiratory waveform, morphology, etc.

In some examples, depending on the particular angle at which the device and sensor are implanted in a particular patient, and/or depending on the particular sleeping position in which the patient is arranged, the inspiration identified from the sensed respiratory waveform may have a positive slope or may have a negative slope. In some such examples, the positive slope or the negative slope of the inspiratory phase may sometimes be referred to as a polarity of the slope of the inspiratory phase. It will be understood that in accounting for the particular slope of the inspiratory phase, the slope of the other phases of the respiratory cycle will be accounted for as well. It is noted that the slope of the inspiratory phase may be identified as a positive slope or a negative slope without determining a value of the slope.

With these features in mind regarding the slope of the inspiratory phase of the respiratory waveform signal, at least some of the example methods and/or devices of the present disclosure may accurately capture and determine respiratory information regardless of how the patient may be moving in space, e.g., regardless of the direction of the sensor rotation in space or regardless of orientation of the patient (including the sensor) with respect to gravity. At least some of the example methods and/or devices of the present disclosure may accurately capture and determine respiratory information without isolating a gravity vector from a sensor signal. Accordingly, the example methods and/or devices may produce accurate, reliable determination of respiration information.

Accordingly, at least some example methods comprise implanting sensor 104A (including sensing element 122A) in a manner to maximize sensitivity of the AC component of the sensed acceleration signal by establishing an orientation (e.g., YR1) of sensing element 122A which is closest to being generally perpendicular to the gravity vector G, for at least some body positions such as a common sleep position (e.g., generally horizontal). In some situations, the sensor 104A (including sensing element 122A) may be implanted in a position in which the sensitivity of the AC component of the sensed acceleration signal is not maximized but which is sufficient to effectively and reliably determine respiration information based on sensed rotational movement at a first portion of a chest wall (or other physiologic location as described below). In some such examples, a sufficient sensitivity of the AC component of the sensed acceleration signal may comprise having an adequate signal-to-noise ratio.

With further regard to the observation that the device (including the acceleration sensor(s)) may be implanted at various particular orientations (e.g., angles) which are not parallel to an ideal reference orientation (e.g., superior-inferior), it will be understood that in some examples, the example methods/devices determine the respiration information (e.g., using acceleration-based sensing of rotational movement of a portion of a chest wall, etc.) without calibrating the measured inclination angle signal (of the acceleration sensor) relative to any difference between the ideal reference orientation (e.g., superior-inferior) and the actual implant orientation. However, in some examples, such calibration may be performed and/or such differences may be considered in using the sensed information.

As noted elsewhere, in at least some examples, determining respiration information based on acceleration sensing of rotational movement (of a portion of the chest wall) does not depend on the sensor having an ideal implant orientation (e.g., with respect to the gravity vector or with respect to the patient's body), does not depend on knowing the actual implant orientation (e.g., with respect to the gravity vector or with respect to the patient's body), and/or does not depend on accounting for differences between the ideal implant orientation and the actual implant orientation.

In some later example implementations, a sensor comprises multiple sensing elements such that the example methods may comprise determining which of the multiple sensing elements has an orientation which is closest to being generally perpendicular to the gravity vector, and therefore which may provide the most sensitivity and effectiveness in sensing respiratory information. In some such examples, the multiple sensing elements may be oriented orthogonally relative to each other or may be oriented at other angles (e.g., 45 degrees) relative to each other.

With further reference to at least FIG. 3, in some examples, the term "generally perpendicular" may comprise the first angular orientation YR1 being at some angle relative to the gravity vector G (e.g., 85, 86, 87, 88, 89, 91, 92, 93, 94, 95 degrees) which varies slightly from an exactly perpendicular angle (e.g., 90 degrees) relative to the gravity vector G. Moreover, as noted above and/or further described below, the effectiveness of measuring respiration by changes in the inclination angle ($\Omega$) between the first and second orientations (YR1, YR2) does not strictly depend on the first angular orientation YR1 being exactly perpendicular to the gravity vector G.

However, the first angular orientation YR1 may be at angles other than generally perpendicular relative to the gravity vector (G), such as in example implementations in which the first angular orientation YR1 of a sensing element (e.g., 122A) is positioned to be about 135 degrees relative to the gravity vector G (i.e., 135 degrees to an anterior-posterior (A-P) orientation of patient's body). In some such examples, the second angular orientation YR2 of sensing element 122A would still extend at an angle ($\Omega$) relative to the first angular orientation YR1, with it being understood that angle ($\Omega$) varies according to the variances in respiration of the patient which occur in normal breathing, forced breathing, and/or disordered breathing, as previously described. As further described later, establishing the first orientation YR1 at angles other than 135 degrees are contemplated as well.

As further described later, in noise model parameter 870 in FIG. 16E the example device(s) and/or example method(s) may perform such measurements in a manner to exclude (e.g., filter) measurements of gross body motion, measurement noise, muscle noise, cardiac noise, other noise, etc. such that the remaining sensed or measured acceleration signal is primarily representative of movement of at least a portion of the chest wall 102. In some such examples, the measured acceleration signal is representative solely of movement of the chest wall 102. In particular, in some such examples, the measured acceleration signal corresponds to rotational movements of at least a portion of the chest wall 102 as sensed by sensor 104A (B1 in FIG. 2) caused by and/or occurring during breathing.

With at least these examples in mind, an example method (and/or example device) is described in association with FIGS. 5A-5B, which comprises at least some of substantially the same features and attributes as, and/or an example implementation of, the examples described in association with at least FIGS. 1A-4.

FIGS. 5A and 5B are diagrams including graphs schematically representing filtered acceleration signals 142A and 142B sensed via a sensor, such as sensing element 122A in FIG. 3, having two different orientations (e.g., due to patient body position), respectively. The filtered acceleration signal 142A of FIG. 5A has a similar orientation as filtered acceleration signal 142 of FIG. 4, while filtered acceleration signal 142B of FIG. 5B has the opposite orientation from filtered acceleration signal 142A of FIG. 5A. As shown in FIGS. 5A and 5B, signals 142A and 142B correspond to a respiratory waveform exhibited through several respiratory cycles during breathing. Each respiratory cycle 143 comprises an inspiratory phase 144A, an expiratory active phase 144B, and an expiratory pause phase 144C. Each respiratory cycle 143 comprises a duration R, which may exhibit breath-to-breath variability. In FIG. 5A, the slope of the expiratory phase 144B is negative as indicated at 155A, and in FIG. 5B, the slope of the expiratory phase 144B is positive as indicated at 155B.

The graphs of FIGS. 5A and 5B include a signal midpoint (e.g., zero crossing) as indicated at 150 and a current time as indicated at 151. The signal midpoint 150 may be provided by a threshold of a bandpass filtered signal, such that zero crossings of the sensor signal indicate the midpoints of each respiratory phase. In some examples, a zero crossing refers to the portion of the respiratory waveform (obtained via a filtered accelerometer signal) at which the amplitude A changes from (e.g., crosses over) a negative amplitude to a positive amplitude (or vice versa) with the amplitude having a value of zero at the crossing point. In some examples, the midpoint may sometimes be referred to as a midpoint of a slope of an inspiratory phase 144A or a midpoint of a slope of an expiratory phase 144B of the respiratory waveform. The bandpass filter may be auto-centering, such that the signal midpoint 150 is maintained despite patient movement that results in changes to the sensor signal. As shown in FIGS. 5A and 5B, the midpoint of each expiratory phase ($T_{E,MID}$) 152 occurs at the zero crossing of each expiratory phase, and the midpoint of each inspiratory phase ($T_{I,MID}$) 153 occurs at the zero crossing of each inspiratory phase. The detection of zero crossings optionally includes hysteresis to prevent false triggering on noise. The hysteresis threshold may be determined by one or more of a: fixed threshold, fraction of recent N peak to peak values (where "N" is an integer), fraction of signal RMS value, dynamic threshold with linear or exponential decay, and time duration since the previous zero crossing.

In addition, a respiratory period may be calculated based on the midpoints of expiratory phases and/or inspiratory phases by calculating the difference between each pair of subsequent $T_{EMID}$ events and/or each pair of subsequent $T_{I,MID}$ events. In other words, in one example, the respiratory period equals the midpoint ($T_{I,MID}$) of an inspiratory phase minus the midpoint ($T_{I,MID}$) of an immediately previous inspiratory phase or equals a running average of the midpoint ($T_{I,MID}$) of an inspiratory phase minus the midpoint ($T_{I,MID}$) of an immediately previous inspiratory phase for N previous respiratory periods, where "N" is an integer number of respiratory periods. In another example, the respiratory period equals the midpoint ($T_{E,MID}$) of an expiratory phase minus the midpoint ($T_{E,MID}$) of an immediately previous expiratory phase or equals a running average of the midpoint ($T_{E,MID}$) of an expiratory phase minus the midpoint ($T_{E,MID}$) of an immediately previous expiratory phase for N previous respiratory periods. A respiratory rate may be calculated as 1 divided by the calculated respiratory period or 1 divided by a running average of N previous respiratory periods. A duration of an expiratory to inspiratory half cycle may be calculated as the midpoint ($T_{I,MID}$) of an inspiratory phase minus the midpoint ($T_{E,MID}$) of the immediately previous expiratory phase or calculated as a running average of the midpoint ($T_{I,MID}$) of an inspiratory phase minus the midpoint ($T_{E,MID}$) of the immediately previous expiratory phase over N previous respiratory periods. A duration of an inspiratory to expiratory half cycle may be calculated as the midpoint of an expiratory phase ($T_{E,MID}$) minus the midpoint ($T_{I,MID}$) of the immediately previous inspiratory phase or calculated as a running average of the midpoint of an expiratory phase ($T_{E,MID}$) minus the midpoint ($T_{I,MID}$) of the immediately previous inspiratory phase over N previous respiratory periods. A midpoint inspiration to expiration (I/E) ratio may be calculated by dividing the duration of the inspiratory to expiratory half cycle by the duration of the expiratory to inspiratory half cycle.

Based on the midpoint of one or more previous expiratory phases and/or one or more previous inspiratory phases, the midpoint of a future inspiratory phase ($PT_{I,MID}$) 156 may be predicted. Based on the predicted midpoint of the future inspiratory phase, a duration D of a stimulation period (e.g., continuous pulsed stimulation) indicated at 157 may be determined. The stimulation period 157 may begin at the beginning of the inspiratory phase or just prior to the inspiratory phase. The stimulation period 157 may end at the end of the inspiratory phase or just after the inspiratory phase. It will be understood that the stimulation period 157 may be followed by a non-stimulation period, as part of a stimulation protocol of a series of consecutive stimulation cycles, with each stimulation cycle including a stimulation period and a non-stimulation period. As apparent from the present context, each stimulation period (of a stimulation cycle) is synchronized relative to a particular portion of the respiratory waveform, e.g., inspiratory phase, according to the various examples described below and throughout examples of the present disclosure.

In one example, the stimulation period 157 is started relative to the predicted midpoint $PT_{I,MID}$ by a first predetermined (e.g., fixed) interval, such as a fixed time period measured in seconds and/or milliseconds. In other examples, the stimulation period 157 is started an amount of time prior to the predicted midpoint $PT_{I,MID}$ computed as a percentage of the respiratory period. For example, the stimulation period 157 may start about 2 seconds prior to the predicted midpoint, where 2 seconds comprises 50% percent of a respiratory period of 4 seconds. In other examples, the stimulation period 157 is started an amount of time prior to the predicted midpoint $PT_{I,MID}$ computed as a percentage of the respiratory rate plus a predetermined (e.g., fixed) amount of time (e.g., 100 ms, 200 ms, etc.). In other examples, the stimulation period 157 is started an amount of time prior to the predicted midpoint $PT_{I,MID}$ computed as a percentage of a duration of the expiratory to inspiratory half cycle (e.g., as a percentage of midpoint ($T_{I,MID}$) of an inspiratory phase minus the midpoint ($T_{E,MID}$) of the immediately previous expiratory phase). For example, the stimulation period 157 may start about 1 second prior to the predicted midpoint, where 1 second comprises 33% percent of a duration of the expiratory to inspiratory half cycle of 3 seconds. In other examples, the stimulation period 157 is started an amount of time prior to the predicted midpoint $PT_{I,MID}$ computed as a percentage of a duration of the inspiratory to expiratory half cycle (e.g., as a percentage of the midpoint of an expiratory phase ($T_{E,MID}$) minus the midpoint ($T_{I,MID}$) of the immediately previous inspiratory phase). For example, the stimulation period 157 may start about 1 second prior to the predicted midpoint, where 1 second comprises 66% percent of a duration of the expiratory to inspiratory half cycle of 1.5 second. In other examples, the stimulation period 157 is started an amount of time prior to the predicted midpoint $PT_{I,MID}$ computed as a percentage of the respiration period (R). For example, the amount of time may comprise about 2 seconds, which is about 50 percent of a respiratory period (R) of about 4 seconds.

In one example, the stimulation period 157 ends after the predicted midpoint $PT_{I,MID}$ by a second predetermined (e.g., fixed) interval as previously described, such as a fixed time period measured in seconds and/or milliseconds. In other examples, the stimulation period 157 ends after the predicted midpoint $PT_{I,MID}$ by an amount of time computed as a percentage of the respiratory rate. In other examples, the stimulation period 157 ends after the predicted midpoint $PT_{I,MID}$ by an amount of time computed as a percentage of the respiratory rate plus a predetermined amount of time. In other examples, the stimulation period 157 ends after the predicted midpoint $PT_{I,MID}$ by an amount of time computed as a percentage of a duration of the expiratory to inspiratory half cycle. In other examples, the stimulation period 157 ends after the predicted midpoint $PT_{I,MID}$ by an amount of time computed as a percentage of a duration of the inspiratory to expiratory half cycle. In other examples, the stimulation period 157 ends after the predicted midpoint $PT_{I,MID}$ by an amount of time computed as a percentage of the respiration period. In yet other examples, the stimulation period 157 may start and/or end based on a combination of any of the above examples. In some of these examples, calculating a stimulation period (e.g., duration, start time, stop time) also may be performed without using a respiratory period in some examples or without using a respiratory rate in some examples.

In some examples, via the above-described arrangement in association with at least FIGS. 5A-5B in which midpoints of respiratory phases (e.g., inspiratory, expiratory) may be used to calculate a respiratory period and/or predict a midpoint of a future inspiratory phase ($PT_{I,MID}$), such calculations, predictions, etc. may be performed without identifying an onset of expiration. Similarly, calculation of a stimulation period (e.g., duration, start time, stop time) also may be performed without identifying an onset of expiration.

FIGS. 5C and 5D are diagrams including graphs schematically representing filtered acceleration signals 142A and 142B sensed via a sensor, such as sensing element 122A in FIG. 3, having two different orientations (e.g., due to patient body position), respectively. The filtered acceleration signal 142A of FIG. 5C has a similar orientation as filtered acceleration signal 142 of FIG. 4, while filtered acceleration signal 142B of FIG. 5D has the opposite orientation from filtered acceleration signal 142A of FIG. 5C. As shown in FIGS. 5C and 5D, signals 142A and 142B correspond to a respiratory waveform exhibited through several respiratory cycles during breathing. Each respiratory cycle 143 comprises an inspiratory phase 144A, an expiratory active phase 144B, and an expiratory pause phase 144C. In FIG. 5C, the slope of the expiratory phase 144B is negative as indicated at 155A, and in FIG. 5D, the slope of the expiratory phase 144B is positive as indicated at 155B.

With this in mind, in some examples an example method (and/or example device) associated with FIGS. 5C-5D may comprise at least some of substantially the same features and attributes as described for FIGS. 5A-5B, except as noted otherwise below. In one aspect, in a manner similar to FIGS. 5A and 5B, FIGS. 5C and 5D also depict peaks 148 and troughs 146.

In this example, the graphs of FIGS. 5C and 5D include a non-midpoint threshold as indicated at 149 and a current time as indicated at 151. As shown in FIGS. 5C and 5D, the non-midpoint threshold 149A is crossed by each expiratory phase 144B and each inspiratory phase 144A. In one aspect, the crossings of the non-midpoint threshold (e.g., 149A) by various portions of the respiratory waveform may occur at any point between a peak 148 and a trough 146 (or vice versa) while excluding the peak 148 and the trough 146. It will be further understood that the particular location of non-midpoint threshold 149A between the peaks 148 and troughs 146 as shown in FIGS. 5C-5D is just one example, and that the non-midpoint threshold may comprise other locations such as, but not limited to, locations 149B and 149C in FIGS. 5C and 5D, respectively. The crossings associated with the non-midpoint threshold 149 may sometimes be further referred to as non-peak, non-trough, non-midpoint crossings and/or as non-midpoint intermediate crossings (e.g., intermediate between a peak and trough or vice versa).

Based on the non-midpoint threshold crossings of one or more previous expiratory phases and/or one or more previous inspiratory phases, the crossing of the non-midpoint threshold 149 of a future inspiratory phase may be predicted. Based on the predicted crossing of the non-midpoint threshold of the future inspiratory phase, a stimulation duration indicated at 157 may be determined. The stimulation may begin at the beginning of the inspiratory phase or just prior to the inspiratory phase. The stimulation may end at the end of the inspiratory phase or just after the inspiratory phase.

In some examples, filtered acceleration signals 142A and 142B may include inflection points and/or curvature of non-peak/non-trough locations, as indicated for example at 147, that may be used to identify each inspiratory phase and/or each expiratory phase since the expiratory phase may have extra inflection point(s) versus the inspiratory phase due to the longer time spent in the expiratory phase (e.g., due to an expiratory pause 144C) prior to the inspiratory phase.

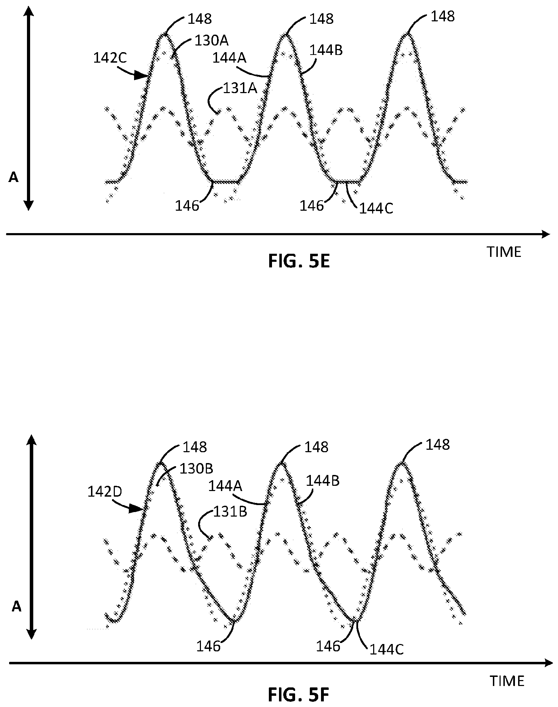

The following FIGS. 5E-5G illustrate example methods (and/or devices) for determining the polarity (i.e., orientation) of a filtered acceleration signal corresponding to a respiratory waveform.

FIG. 5E is a diagram including a graph schematically representing a reference signal 142C, which may be fitted to a filtered acceleration signal. A filtered acceleration signal (e.g., 142A of FIG. 5A) has a similar orientation and shape as reference signal 142C of FIG. 5E. As shown in FIG. 5E, reference signal 142C corresponds to a respiratory waveform exhibited through several respiratory cycles during breathing. Reference signal 142C corresponds to peaks 148, troughs 146, inspiratory phases 144A, expiratory active phases 144B, and expiratory pause phases 144C of a respiratory waveform. By fitting a filtered acceleration signal to reference signal 142C, the filtered acceleration signal polarity may be determined. In this example, the polarity of the filtered acceleration signal is such that the slope of the inspiratory phase 144A is positive, and the slope of the expiratory phase 144B is negative.

Reference signal 142C is a composite signal comprising a combination of a sinusoid 130A at a fundamental frequency and a sinusoid 131A at the second harmonic. The reference signal 142C may approximate a filtered acceleration signal, since the second harmonic adds to the fundamental at the peak 148, making the peak sharper, and subtracts from the fundamental at the trough 146, making the trough flatter. The peak 148 corresponds to the transition between inspiration 144A and expiration 144B, while the flat trough 146 corresponds to the expiration pause 144C. Thus, a filtered acceleration signal may be decomposed into its fundamental and second harmonic components. Where the fundamental and second harmonic components strengthen each other indicates the transition from inspiration 144A to expiration 144B, and where the fundamental and second harmonic components weaken each other indicates the expiration pause 144C.

FIG. 5F shows a reference signal 142D including a composite of a sinusoid 130B and second harmonic 131B in which the phase of the second harmonic has been modified (e.g., shifted). This modification to the phase of the second harmonic adds slope to the trough 146 for the expiration pause 144C, but the difference in shape between the peak 148 and the trough 146 is still evident. Filtered acceleration signals from different patients may exhibit different relative phases between the fundamental and the second harmonic components, so this phase parameter of the second harmonic may be determined during device setup and used during operation to better determine the filtered acceleration signal polarity.

A least squares fitting may be used to find the coefficients $c_1$, $s_1$, $c_2$, $s_2$, so that $c_1 \cos(\omega t) + s_1 \sin(\omega t) + c_2 \cos(2\omega t) + s_2 \sin(2\omega t)$ best approximates the filtered acceleration signal (which may also be referred to as a breathing signal $b(t)$). The phase of the fundamental component is $\varphi_1 = \arg(c_1 + is_1)$ and the phase of the second harmonic component is $\varphi_2 = \arg(c_2 + is_2)$ where arg is the argument of a complex number (angle between real axis and current position in complex plane). The polarity of the signal can be determined from the difference in phase $\Delta\varphi = \varphi_2 - 2\varphi_1$. The effectiveness of this method improves when the frequency is stable (i.e., the acceleration signal period does not change much from breath to breath). The difference in phase may be used to distinguish inspiration from expiration, where a phase difference falling within a certain range indicates the phase in the respiratory cycle. In some examples, the range may be a fixed value, while in other examples the range may be configured for each patient.

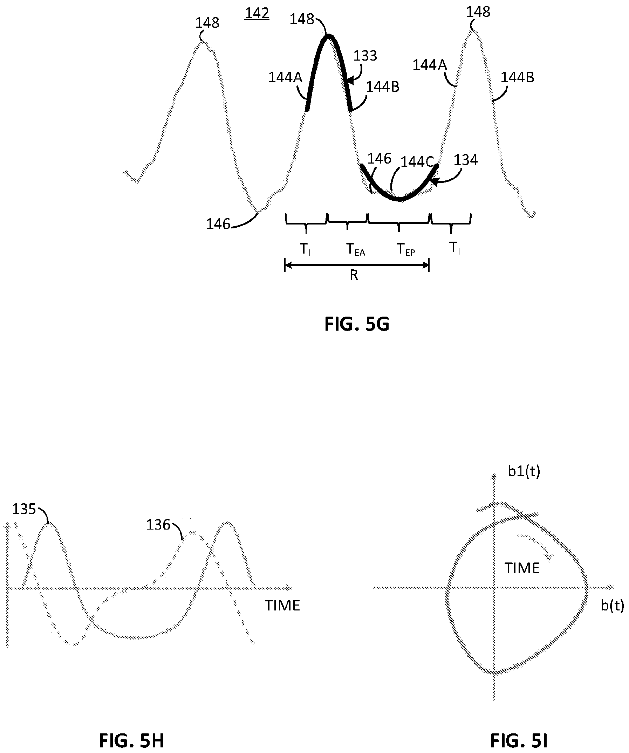

FIG. 5G is a diagram including a graph schematically representing fitting example peaks 148 and troughs 146 of a filtered acceleration signal 142 to reference signals. In this example, coefficients of a polynomial 133 fit to a window of data around the peak 148 of the filtered acceleration signal 142 may be compared to coefficients of a polynomial 134 fit to a window of data around the trough 146 of the filtered acceleration signal 142. As shown in FIG. 5G, parabolas (e.g., quadratic polynomials) 133 and 134 are fit to one peak 148 and one trough 146 of the filtered acceleration signal 142. Comparing the absolute value of the coefficient of the quadratic term of these polynomials 133 and 134 is equivalent to comparing the curvature of the filtered acceleration signal 142 at the peak 148 to the curvature of the filtered acceleration signal 142 at the trough 146. The peak or trough having the smaller curvature may be identified as the inspiration 144A to expiration 144B transition, while the peak or trough having the larger curvature may be identified as the expiratory pause 144C.

For each method for determining the polarity of the filtered acceleration signal (e.g., breathing signal $b(t)$) described above, processing signals over multiple breaths improves the signal-to-noise ratio to the point where the subtle features that determine polarity can be reliably resolved. This may be achieved by buffering signals and/or calculating the quantities of interest via filters.

Some methods for determining the next start of inspiration also rely on estimates of the breathing rate, i.e., the dominant frequency $\omega$ of $b(t)$. One method for estimating $\omega$ may include, in the least-squares fitting of $c_1$, $s_1$, $c_2$, $s_2$ described above, also including a (non-linear) least-squares fitting of $\omega$. Another method for estimating $\omega$ may consider the time separation between consecutive peaks, with some method being used to ignore minor peaks. For example, only the highest positive peak is considered within a window of a certain size, and likewise only the lowest negative peak is considered within a window of a certain size.

FIG. 5H is a diagram including a graph schematically representing an example filtered acceleration signal $b(t)$ indicated at 135 and the estimated rate-of-change $b1(t)$ indicated at 136 of the example filtered acceleration signal 135. FIG. 5I is a diagram including a graph schematically representing a locus of the estimated rate-of-change $b1(t)$ 136 of the example filtered acceleration signal versus the example filtered acceleration signal $b(t)$ 135 of FIG. 5H. Another method for estimating $\omega$ may consider the locus of the points $(b(t), b1(t))$, where $b1(t)$ relates to the rate of change of $b(t)$, as illustrated in FIG. 5H. The $b1(t)$ can be estimated with a suitable filter, e.g., a combination of a differentiating filter and a low pass filter. The frequency $\omega$ can be estimated from the ratio of the norm of $b1(t)$ to the norm of $b(t)$, e.g., RMS (a.k.a., L2 norm). If $b1(t)$ is the Hilbert transform of $b(t)$ (again, estimated with a suitable filter), then the frequency can be estimated from the rate of change of the instantaneous phase $\varphi(t) = \arg(b(t) + ib1(t))$, after unwrapping $\varphi(t)$.

Various steps in a motion processing chain (e.g., as described below with reference to at least FIGS. 16A-16F) may result in delay, i.e., so that $b(t)$ relates to the actual breathing pattern at time $t - \Delta t$ for some delay $\Delta t$. This delay may be characterized and compensated for by a number of methods. For example, a fixed delay may be used, based on the response of the motion processing chain to a typical breathing signal. In some examples, the delay may be within a range of about 0.15-0.25 seconds for 89 percent of the datasets, such that a fixed delay of about 0.2 seconds would be adequate in most cases. In other examples, the delay can be applied as a function of the current estimate of the breathing frequency. In yet other examples, a patient-specific delay can be applied, based on collating a histogram of delays calculated using the method described above and using the most common delay.

With at least these examples in mind, example methods are described in association with FIGS. 6A-6B, which comprise at least some of substantially the same features and attributes as, and/or an example implementation of, the examples described in association with at least FIGS. 1A-5B.

FIG. 6A is a flow diagram illustrating one example of a method 158 for identifying the slope of an expiratory phase. In one example, method 158 may be implemented by control portion 14A of FIG. 1A or control portion 14B of FIG. 1B. At 158A, the control portion may determine the median of data points of the sensor signal between a current midpoint (e.g., $T_{I,MID}$ at current time 151 in FIGS. 5A and 5B) and at least two previous midpoints (e.g., as indicated at 154 in FIGS. 5A and 5B), such as a multiple N of two previous midpoints corresponding to N complete respiratory cycles. In some examples, more than two previous midpoints may be used, such as 4, 6, 8, etc. In other examples, many (e.g., 11 or more) previous midpoints may be used to determine the median, and deviation from a multiple N of two previous midpoints corresponding to N complete respiratory cycles is acceptable (i.e., an odd number of half cycles may be used). At 158B, the control portion may identify the slope of the expiratory phase as negative (e.g., as indicated at 155A in FIG. 5A) in response to the median being less than 0. At 158C, the control portion may identify the slope of the expiratory phase as positive (e.g., as indicated at 155B in FIG. 5B) in response to the median being greater than 0.

In other examples, the control portion may determine the root-mean-square (RMS) of the data points of the sensor signal above the signal midpoint (e.g., 150 of FIGS. 5A and 5B) and the RMS of the data points of the sensor below the signal midpoint between a current midpoint (e.g., $T_{I,MID}$ at current time 151 in FIGS. 5A and 5B) and at least two previous midpoints (e.g., as indicated at 154 in FIGS. 5A and 5B), such as a multiple N of two previous midpoints corresponding to N complete respiratory cycles. The control portion may identify the slope of the expiratory phase as negative (e.g., as indicated at 155A in FIG. 5A) in response to the RMS of the data points above the signal midpoint being less than the RMS of the data points below the signal midpoint. The control portion may identify the slope of the expiratory phase as positive (e.g., as indicated at 155B in FIG. 5B) in response to the RMS of the data points above the signal midpoint being greater than the RMS of the data points below the signal midpoint.

The sign of the median indicates the sign of exhalation slope since respiration typically has an I/E ratio that is less than 1.0. Using the median is less sensitive to noise than using the I/E ratio directly, because the I/E ratio would require accurately finding the peaks and troughs in a noisy signal. The steeper slope at the zero crossing is less sensitive to noise than the flat tops/bottoms of a peak/trough based method.

For a small subset of patients, the identification of each inspiratory phase and expiratory phase may be consistently and repeatedly incorrect, such that for this subset of patients the identified inspiratory phase is actually an expiratory phase and the identified expiratory phase is actually an inspiratory phase. To address this subset of patients, in some examples, the control portion 148 (FIG. 1B) may include an invert function. With the invert function active, method 158 of FIG. 6A is inverted such that at 158B, the control portion may identify the slope of the inspiratory phase as negative in response to the median being less than 0; and at 158C, the control portion may identify the slope of the inspiratory phase as positive in response to the median being greater than 0. Thus, with reference to FIGS. 5A-5D, with the invert function active, an expiratory phase may be identified at 144A, and an inspiratory phase may be identified at 144B.

FIG. 6B is a flow diagram illustrating one example of a method 159 for identifying the slope of an inspiratory phase. In one example, method 159 may be implemented by control portion 14A of FIG. 1A or control portion 14B of FIG. 1B. At 159A, the control portion may determine the median of data points of the sensor signal between a current midpoint (e.g., $T_{I,MID}$ at current time 151 in FIGS. 5A and 5B) and at least two previous midpoints (e.g., as indicated at 154 in FIGS. 5A and 5B), such as a multiple N of two previous midpoints corresponding to N complete respiratory cycles. In some examples, more than two previous midpoints may be used, such as 4, 6, 8, etc. In other examples, many (e.g., 11 or more) previous midpoints may be used to determine the median, and deviation from a multiple N of two previous midpoints corresponding to N complete respiratory cycles is acceptable (i.e., an odd number of half cycles may be used). At 159B, the control portion may identify the slope of the inspiratory phase as positive (e.g., as shown in FIG. 5A) in response to the median being less than 0. At 159C, the control portion may identify the slope of the inspiratory phase as negative (e.g., as shown in FIG. 5B) in response to the median being greater than 0.

In other examples, the control portion may determine the root-mean-square (RMS) of the data points of the sensor signal above the signal midpoint (e.g., 150 of FIGS. 5A and 5B) and the RMS of the data points of the sensor below the signal midpoint between a current midpoint (e.g., $T_{I,MID}$ at current time 151 in FIGS. 5A and 5B) and at least two previous midpoints (e.g., as indicated at 154 in FIGS. 5A and 5B), such as a multiple N of two previous midpoints corresponding to N complete respiratory cycles. The control portion may identify the slope of the inspiratory phase as positive (e.g., as shown in FIG. 5A) in response to the RMS of the data points above the signal midpoint being less than the RMS of the data points below the signal midpoint. The control portion may identify the slope of the inspiratory phase as negative (e.g., as shown in FIG. 5B) in response to the RMS of the data points above the signal midpoint being greater than the RMS of the data points below the signal midpoint.

With the invert function active, method 159 of FIG. 6B is inverted such that at 159B, the control portion may identify the slope of the expiratory phase as positive in response to the median being less than 0; and at 159C, the control portion may identify the slope of the expiratory phase as negative in response to the median being greater than 0.

FIGS. 7A, 7B, and 8 are diagrams which schematically represent an example method and/or example sensor 204 which may comprise three sensing elements 122A (Y), 162 (Z), 164 (X) arranged orthogonally relative to each other. In some examples, the sensor 204 (including at least sensing element 122A) comprises at least some of substantially the same features and attributes as sensor 104A previously described in association with at least FIGS. 2-4 in which just one sensing element 122A (Y) is present. However, as shown in FIGS. 7A-7B, in addition to sensing element 122A (Y), in some examples sensor 204 also may comprise acceleration sensing element 162 having orientation Z (Z-axis) which is perpendicular to sensing element 122A. As implanted, this Z-axis orientation is generally perpendicular to a superior-inferior (S-I) orientation of the chest wall 102A, and is generally parallel to an anterior-posterior (A-P) orientation of the chest wall 102A.

Meanwhile, as shown in FIG. 7B, in addition to comprising sensing element 122A (Y), in some examples sensor 204 also may comprise acceleration sensing element 164, having orientation X (X-axis) which is generally perpendicular to sensing element 122A. As implanted, this X-axis orientation is generally perpendicular to a superior-inferior (S-I) orientation of the chest wall 102A, and generally perpendicular to an anterior-posterior (A-P) orientation of the chest wall 102A. In some such examples, sensing element 164 may sense rotational movement of chest wall 102A (as represented by directional arrow B5) in a plane defined by the anterior-posterior orientation (A-P) and by the lateral-medial orientation (L-M), according to changes in an inclination angle (as represented via directional arrow B4) of sensing element 164. Each of the respective sensing elements 162 (Z), 164 (X) may provide additional sensing of rotational movement of the chest wall 102A to provide further respiration information. With further reference to FIGS. 7A, 7B, and 8, in some examples, sensor 204 may comprise all three sensing elements 122A (Y), 162 (Z), and 164 (X).

As schematically represented in the diagram 250 of FIG. 8, the sensed acceleration signal information from each of the three sensing elements 162, 122A, 164 of sensor 204 may be combined to provide composite rotational change information (252). In some examples, the composite rotational change information 252 may sometimes be referred to as a virtual vector representing the overall rotational movement (e.g., according to at least two orthogonal axes) caused by breathing. In some such examples, the composite rotational change information 252 corresponds to sensing the AC component of the multi-dimensional acceleration vector (e.g., a virtual vector) with respect to gravity.

The virtual vector can be determined from the sensed acceleration signal information from each of the three sensing elements 162, 122A, and 164 of sensor 204 using various methods. In one example, Principal Component Analysis (PCA) may be performed on the sensed acceleration signals to identify the composite of the sensed acceleration signals having the greatest variation. That is, the sensed acceleration signals are arranged in a $N \times 3$ matrix $F$, which is decomposed into an $N \times 3$ matrix $U$ of orthonormal column vectors, a $3 \times 3$ diagonal matrix $S$ of singular values and a $3 \times 3$ matrix V of orthonormal column vectors: $F = USV^T$. Assuming $S_{1,1}$ is the largest singular value, the first column of V corresponds to the breathing axis $B$ (i.e., the virtual vector or component of the signal which most strongly relates to breathing) and the first column of $U$, multiplied by $S_{1,1}$, corresponds to the breathing signal b. That is, $B$ is the axis along which the sensed acceleration signals show the most variance, and b is the component of the sensed acceleration signals along this axis. The entries of b are indexed by time, so the breathing signal may be written as $b(t)$ (i.e., the composite rotational change information).

Furthermore, the method can be performed in a computationally efficient way. One way is to perform eigenanalysis of the symmetric $3 \times 3$ matrix $C = F^T F$, which requires less computation than PCA of $F$ when $N$ is large. This gives a $3 \times 3$ matrix $V$ of eigenvectors, identical to $V$ described above, and 3 eigenvalues. The column of $V$ corresponding to the largest eigenvalue is the breathing axis B and the breathing signal is calculated as $b = FB$. Another computationally efficient method that provides an iterative approximation to $B$ and $b$ is the power method. This is applicable because the power method only determines the largest principal component of $F$.

In some examples, at least two of the three orthogonally-arranged sensing elements may be used to perform determination of composite rotational movement and therefore respiration information at least based on an AC component of a multi-dimensional acceleration vector produced by the n single-axis sensing elements.

In some such examples associated with FIG. 8, the virtual vector corresponding to the composite rotational change (252) may exhibit higher sensitivity to respiration than any single physical measurement axis of a physical sensing element 122A (Y), sensing element 162 (Z), or sensing element 164 (X). In some such examples, the virtual vector (252) may exhibit a higher signal-to-noise ratio (e.g., signal quality) than any single physical measurement axis, such as single sensing element 122A (Y) or single sensing element 162 (Z) or single sensing element 164 (X) by virtue of combining the signals of the multiple sensing elements.

In some such examples, the virtual vector (e.g., 252) effectively excludes non-physiologic motion of the chest wall. At least some examples of such non-physiologic motion may comprise motion of a vehicle (e.g., car, airplane, etc.)

within which the patient is riding, of patient swinging in a hammock, and the like. Accordingly, determining respiration information via the virtual vector in such example methods and/or devices may produce respiration information which is generally insensitive to non-physiologic motion of the patient.

In some examples, respiration detection may be based on a virtual vector which is a sum of two of the measurement axes from among the three orthogonally-arranged sensing elements. In some examples, respiration detection may be based on a sum of signals from all three orthogonally-arranged sensing elements. In some examples, respiration detection may be determined by looking independently at each of the three measurement axes or from among the three axes. It will be understood that in any of these examples of using one, two, or three measurement axes from a multi-axis accelerometer, the respiration detection is performed relative to an earth gravity vector such that the earth gravity vector is not isolated, not excluded, etc. from the determination of a respiratory waveform, determination of a feature (e.g., midpoint crossing, non-midpoint crossing, etc.) of a respiratory waveform, and so on.

Moreover, respiratory information determinations in at least some examples of the present disclosure are performed without (necessarily) excluding (from the respiratory information determination) one of three axes of a multi-axis accelerometer upon mandatory association (e.g., pairing, matching, etc.) of just one of the three axes with a gravity vector such that just the remaining two axes would be used for the determination of respiratory information (if one of the three axes were excluded).

Accordingly, via at least some examples of the present disclosure, one, some, or all of the measurement axes of a multiple axis accelerometer may be used at any point in time during a sleep period (e.g., treatment period) in a dynamic manner because such examples do not rely on excluding a measurement axis designated in association with a gravity vector. Patients may move frequently during a sleep period. Accordingly, the dynamic, flexible manner in which examples of the present disclosure provide rotational sensing relative to an earth gravity vector and provide determination of respiratory information (e.g., phases, stimulation period, etc.) based on phase midpoints may provide a significantly more robust respiration detection method and/or device.

In some examples, a method and/or device may employ control portion 900 (FIG. 17A) to select the virtual vector (e.g., 252) or select a physical measurement axis from one of the sensing elements 122A, 162, or 164 for use in determining respiration information. In some such examples, the method and/or device may evaluate the robustness of the determined respiration information and automatically convert operation among the virtual vector (e.g., 252 in FIG. 8) and any one of the physical measurement axes (e.g., 122A/Y, 162/Z, 164/X) to consistently use the most robust, accurate signal source in determining respiration information.

In association with the examples of at least FIGS. 7A, 7B, and 8, in some examples the signal-to-noise ratio of a virtual vector and/or physical measurement axis may be enhanced via excluding noise, such as later described in association with at least noise model parameter 870 (FIG. 16E).

In some examples, the above-described measuring of rotational movement (of a portion of a chest wall via acceleration sensing) per sensing element 162 (Z-axis) may be likened to a pitch parameter, measuring rotational movement per sensing element 122A (Y-axis) may be likened to a yaw parameter, and measuring rotational movement per sensing element 164 (X-axis) may be likened to a roll parameter. Because of variances in anatomy from patient to patient, the particular implant orientation, and/or the particular implant location (e.g., front vs. side of the chest), the pitch parameter, yaw parameter, and/or roll parameter may bear a rough or general correspondence to the ideal definition for such respective parameters in which the pitch parameter may correspond to rotational movement of the portion of the chest wall in a first plane defined by an anterior-posterior orientation and by a superior-inferior orientation of the patient's body. Similarly, the yaw parameter may roughly or generally correspond to rotational movement of the portion of the chest wall in a second plane defined by the anterior-posterior orientation and by a lateral-medial orientation of the patients' body. Similarly, the roll parameter may roughly or generally correspond to rotational movement of the portion of the chest wall in a third plane defined by the lateral-medial orientation and by the superior-inferior orientation of the patient's body.

In examples in which the patient's body position corresponds to a primary sleeping position (e.g., generally horizontal), then the magnitude of changes in the AC signal component from rotational movement (B3) of sensing element 162 (Z axis) during breathing will be negligible and the magnitude of changes in the AC signal component from rotation (arrow B4) of sensing element 164 (X axis) during breathing may be relatively small at least compared the magnitude of changes in the AC signal component of sensing element 122A (Y-axis) during breathing (as described in association with FIGS. 2-4).

However, in some example situations the patient's body position may correspond to a secondary or alternate sleep position, such as sitting upright against a support 273 (e.g., ordinary chair, airplane chair, etc.) as shown in FIG. 10 or in a partially reclined position (e.g., torso is 45 degrees from horizontal) against a support 263 (e.g., recliner chair, recliner bed, etc.) which is at angle ($\lambda$) relative to generally horizontal (e.g., floor) as shown in FIG. 9A. In some such examples, such as the partially reclined position in FIG. 9A, at least the respective sensing element 162 (Z axis) may yield significant magnitude of changes in the AC signal component during breathing instead of and/or in addition to sensed changes in the AC signal component of sensing element 122A (Y-axis) during breathing. In this example, the sensing element 122A may comprise a first angular orientation (like YR1 in FIG. 3 for peak expiration) which is 45 degrees ($\sigma$ in FIG. 9B) relative to the gravity vector G (and which is 45 degrees relative to a generally horizontal plane, which typically is a primary sleep position). While the first orientation (e.g., YR1) of the sensing element 122A may not be generally perpendicular to the gravity vector G as in FIG. 3, at the first orientation of 45 degrees (σ in FIG. 9B) relative to the gravity vector G, the acceleration sensing element 122A still exhibits sufficient sensitivity in the AC signal component to produce meaningful measurements in changes of the inclination angle (e.g., Ω in FIG. 3) of sensing element 122A between the first and second orientations (e.g., YR1 and YR2) during breathing to enable determining respiration information.

In this example, the sensing element 162 may comprise a first orientation (like YR1 in FIG. 3) which extends at an angle of 135 degrees (θ in FIG. 9C) relative to the gravity vector G (and which is 45 degrees relative to a generally horizontal plane, which typically is a primary sleep position). While the sensing element 162 may not be generally perpendicular to the gravity vector G (as was sensing element 122A in the example of FIG. 3), at the first orientation of 135 degrees (θ in FIG. 9C) relative to the gravity vector G, the acceleration sensing element 162 exhibits sufficient sensitivity in the AC signal component to produce meaningful measurements in changes of the inclination angle (like Ω in FIG. 3) of sensing element 162 between its first orientation (peak expiration) and second orientation (peak inspiration) during breathing to enable determining respiration information.

In a manner similar to that shown in FIG. 8, the sensed rotational movement from at least the multiple sensing elements (e.g., 162/Z-axis and 122A/Y-axis in FIGS. 9A-9C) may be combined to yield a composite value of sensed rotational movement of sensor 204 in order to produce sensing of a respiratory waveform while the patient is in the partially reclined position.

It will be further understood that, in some examples, the sensing element 164 (X-axis) also may be used in addition to sensing elements 122A, 162 (and in a manner similar to that described for sensing elements 122A, 162 in FIGS. 9A-9C) to provide further sensing by which the determination of respiration information can be made, with the rotational sensing information being combined, similar to that shown in FIG. 8. With this in mind and with further reference to at least the examples of FIGS. 9A-9C and 10, it will be understood that employing a three axis accelerometer (in which the three axes are orthogonally-arranged) will ensure that at least one of the three axes will have an output signal of magnitude sufficient to reliably determine respiration (e.g., based on rotational movement of the sensor in correspondence with rotational movement of a portion of the chest wall during breathing as described in various examples). It is noted that a single three axis accelerometer may be used to determine respiration without using any additional sensing elements, such as a second three axis accelerometer.

It will be understood that in some examples, the particular angle λ of reclination in FIG. 9A may be angles other than 45 degrees, and may be variable over time in some instances, depending on the type and manner of support 263 (e.g., adjustable bed, chair). In some such examples, a determination of respiration information may be based on the particular respective sensing element(s) (e.g., 122A (Y-axis), 162 (Z-axis), 164 (X-axis)) having the orientation(s) closest to being generally perpendicular to the gravity vector G for the particular angle λ at a particular point in time.

Moreover, in this example arrangement of FIGS. 9A-10, if and/or when the patient moves to another sleeping position, such as generally horizontal position (e.g., FIG. 3), then the sensing element 122A (or sensing element 164) may become the sole or primary signal source for detecting respiration in some examples. Accordingly, example arrangements of multiple single-axis acceleration sensing elements in orthogonal relationship to each other may provide robust sensing of respiration which enables adaptability in response to a patient moving among different sleep positions within a single treatment period or among multiple, different treatment periods.

As previously noted, the FIG. 10 schematic represents at least a chest wall 102A of a patient's body in a generally vertically upright position, such as if the patient were sitting on a support 276 with their torso against a vertical support 273. In this example arrangement, both the acceleration sensing elements 162 (Z-axis) and 164 (X-axis) of sensor 204 may have a first orientation which is generally perpendicular (or reasonably close to being generally perpendicular) to gravity vector G, whereas the acceleration sensing element 122A (Y-axis) of sensor 204 has a general orientation which is generally parallel to gravity vector G. Accordingly, for substantially similar reasons presented with respect to FIGS. 3 and 7A-9C, one or both of the sensing elements 162 (Z-axis), 164 (X-axis) may provide the most sensitive sensing elements by which respiration information determination may be performed.

In particular, upon rotational movement of the patient's chest wall 104A during breathing within a treatment period, rotational movement of Z-axis sensing element 162 between a first orientation (e.g., like YR1 in FIG. 3) and a second orientation (e.g., like YR2 in FIG. 3) may be sensed as a range of values of an AC signal component from which a respiratory waveform (including respiratory phase timing/details) may be determined as shown in FIGS. 4-5B. Moreover, rotational movement of X-axis sensing element 164 may provide similar information and may be used to determine respiration information. The respiration information may be determined solely from the Z-axis sensing element 162, solely from the X-axis sensing element 164, or from a combination of information sensed via both of the Z-axis sensing element 162 and the X-axis sensing element 164. While the Y-axis sensing element 122A would generally be expected to produce negligible or minimal respiration information (because of being parallel to the gravity vector G), in some examples, information sensed from Y-axis sensing element 122A may be combined with rotational information sensed via the sensing elements 162, 164.

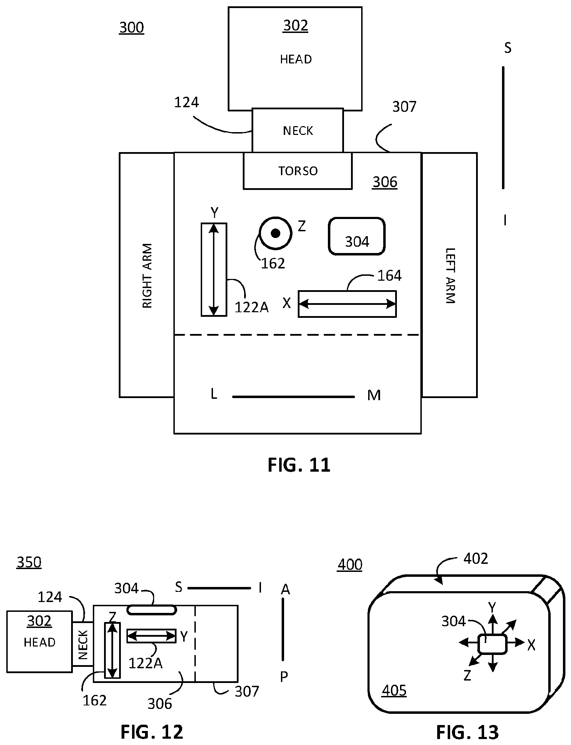

FIG. 11 is a diagram 300 including a front view schematically representing different measurement axes of an example sensor 304 and/or related example method. In some examples, the sensor 304 may comprise at least some of substantially the same features and attributes as the sensors, sensing elements, and related example methods as previously described in association with FIGS. 1A-10. As shown in FIG. 11, sensor 304 is implanted within a wall of chest region 306 of torso 307 below a neck 124 and head 302. The sensor 304 comprises multiple sensing elements 122A (Y-axis orientation), 162 (Z-axis orientation), 164 (X-axis orientation), which may be independent such as three separate single-axis accelerometers, or these sensing elements may be combined into a single arrangement, such as a three-axis accelerometer. FIG. 12 is a diagram 350 including a side view schematically representing the sensor 304 of FIG. 11, highlighting the orientation of the sensing elements 122A, 162.

FIG. 13 is a diagram 400 including an isometric view schematically representing an implantable device 402 comprising an accelerometer-based sensor 304, which may comprise at least some of substantially the same features and attributes as the sensors, sensing elements, and related example methods as previously described in association with FIGS. 1A-12. It will be understood that the sensor (and sensing elements) described in FIGS. 1A-12 may be implemented as being on or within device 402. In some examples, sensor 304 is enclosed within a sealed housing (e.g., can) of the device 402. However, the sensor 304 may be external to the housing 405 of device 402, whether located on the housing or extending from the housing 405 on a lead.

With further reference to FIG. 13, in some examples, device 402 may comprise an implantable device, which includes circuitry and power elements to operate the sensor 304 to sense physiologic phenomenon, such as but not limited to respiration information. In some examples, the circuitry and power may be implemented within or as part of a control portion 900 and/or related portions, elements, functions, parameters, engines, as further described later in association with at least FIGS. 16A-16E. Among other attributes, via the control portion, the device 402 may be used to monitor and/or diagnose physiologic phenomenon, patient conditions (e.g., respiratory health, cardiac health, etc.), with one such patient condition including sleep disordered breathing (SDB). In some examples, device 402 may comprise an implantable pulse generator (IPG), which may implement neurostimulation in association with respiration detection in order to treat sleep disordered breathing and/or other patient health conditions. In some such examples, the device 402 may also sense translational movements of the chest wall and/or associated body tissue in order to sense, monitor, diagnose, etc. the various physiologic phenomenon, patient conditions, etc. whether the sensed translational movement is obtained instead of, or in addition to, the sensed rotational movement of the portion of the chest wall.

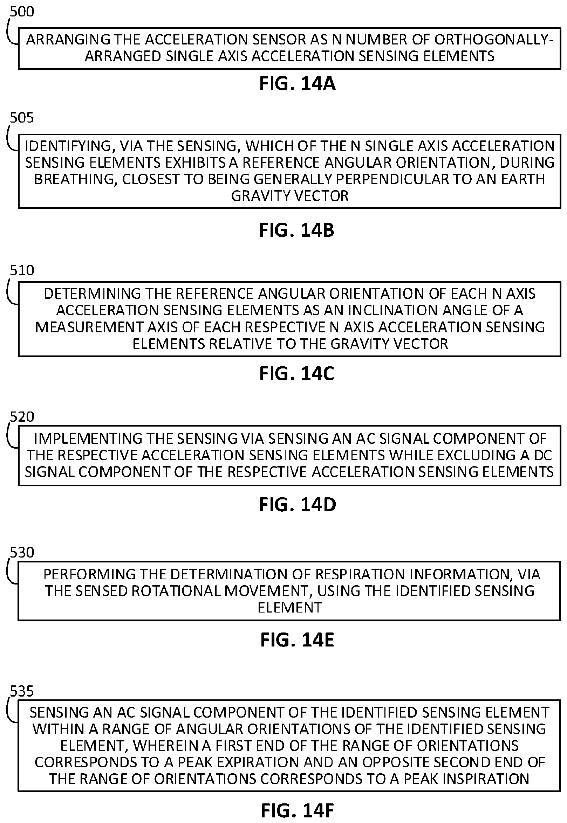

With the examples of FIGS. 1A-13 in mind, it will be understood that in some examples the sensor signal which will be used to determine respiration information may be selected from among multiple sensing elements, such as but not limited to, the individual axis of the three-axis accelerometer. Accordingly, at least some example methods and/or devices as described in association with at least FIGS. 14A-14K further describe such selection.

Accordingly, as shown at 500 in FIG. 14A, some example methods and/or devices for determining respiration information may comprise arranging the acceleration sensor as n number of orthogonally-arranged single axis acceleration sensing elements. As shown at 505 in FIG. 14B, in some examples the method comprises identifying, via the sensing, which of the n single axis acceleration sensing elements exhibits a reference angular orientation, during breathing, closest to being generally perpendicular to the gravity vector. In some such examples, as shown at 510 in FIG. 14C, the method comprises determining the reference angular orientation of each n axis acceleration sensing elements as an inclination angle of a measurement axis of each respective n axis acceleration sensing elements relative to the gravity vector.

In some examples associated with FIGS. 14A-14C, as shown at 520 in FIG. 14D, the method comprises implementing the sensing via sensing a AC signal component of the respective acceleration sensing elements while excluding (or at least minimizing) a DC signal component of the respective acceleration sensing elements.

With reference to the example method in at least 505 in FIG. 14B, the example method may comprise performing the determination of respiration information, via the sensed rotational movement, using the identified sensing element as shown at 530 in FIG. 14E. In some such examples, the method comprises performing the determination, via the sensed rotational movement, comprises using at least two of the acceleration sensing elements.

In some such examples as previously described in FIGS. 14A-14E, one example method comprises, as shown at 535 in FIG. 14F, determining the respiration information comprises sensing an AC signal component of the identified sensing element within a range of angular orientations of the identified sensing element, wherein a first end of the range of orientations corresponds to a peak expiration and an opposite second end of the range of orientations corresponds to a peak inspiration. In some examples, the first end of the range of orientations corresponds to the reference angular orientation.

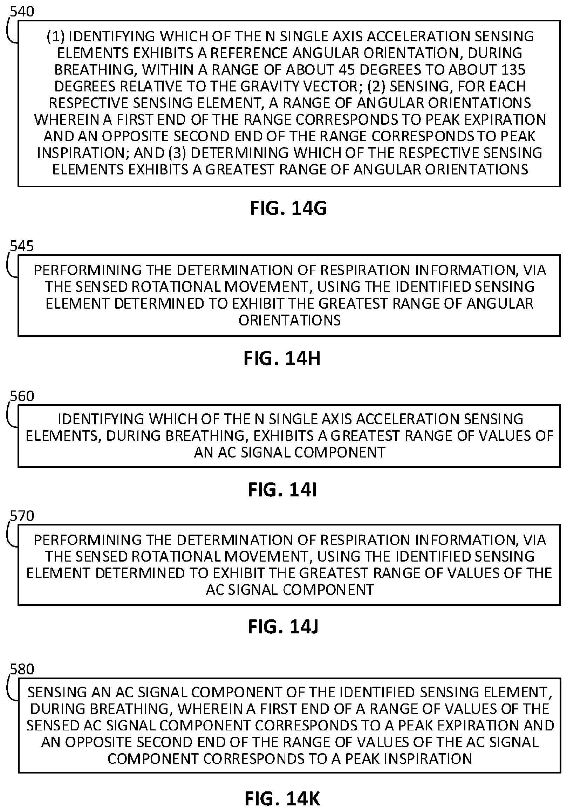

As shown at 540 in FIG. 14G, in some examples of determining respiration information, the method comprises: (1) identifying which of the n single axis acceleration sensing elements exhibits a reference angular orientation, during breathing, within a range of about 45 degrees to about 135 degrees relative to the gravity vector; (2) sensing, for each respective identified acceleration sensing element, a range of angular orientations relative to the gravity vector, wherein a first end of the range of orientations corresponds to a peak expiration and an opposite second end of the range of orientations corresponds to a peak inspiration; and (3) determining which of the identified acceleration sensing elements exhibits a greatest range of angular orientations. In some examples, method 540 further comprises, as shown at 545 in FIG. 14H, performing the determination of respiration information, via the sensed rotational movement, using the identified acceleration sensing element determined to exhibit the greatest range of angular orientations.

In some such examples, such as at 540, the method may comprise performing the determination, via the sensed rotational movement, comprises using all of the identified acceleration sensing elements. In some examples of at least method 500 (FIG. 14A) and the associated aspects in FIGS. 14B-14H, the variable n equals 3.

With further reference to the example method shown at 500 in FIG. 14A, in some examples, as shown at 560 in FIG. 14I, some example methods comprise identifying which of the n single axis acceleration sensing elements, during breathing, exhibits a greatest range of values for an AC signal component. In some such examples, as shown at 570 in FIG. 14J, the method comprises performing the determination of respiration information, via the sensed rotational movement, using the identified acceleration sensing element determined to exhibit the greatest range of values of the AC signal component.

With regard to example methods in at least FIGS. 14I and/or 14J, the example method may comprise determining a sensing signal for each n axis acceleration sensing elements as an inclination angle of a measurement axis of each respective n axis acceleration sensing elements relative to the gravity vector, in a manner similar to that previously shown at 510 in FIG. 14C.

With regard to example methods in at least FIGS. 14I and/or 14J, one example method (as shown at 580 in FIG. 14K) may further comprise determining the respiration information via sensing an AC signal component of the identified sensing element during breathing, wherein a first end of a range of values of the sensed AC signal component corresponds to a peak expiration and an opposite second end of the range of values of the sensed AC signal component corresponds to a peak inspiration.

FIG. 15 is a diagram including a front view of an example device 611 (and/or example method) implanted within a patient's body 600. In some examples, the device 611 may comprise an implantable device 633 such as (but not limited to) an implantable pulse generator (IPG) with device 633 including a sensor 635. In some examples, sensor 635 may comprise a sensor (e.g., 12A, 12B, 104A, 122A, 162, 164, 204, 304, etc.) having at least some of substantially the same features and attributes as previously described in association with at least FIGS. 1A-14K. Via such example sensing arrangements, the device 633 may determine respiration information via sensing rotational movement of the patient's chest wall during breathing, such as but not limited to when in a sleeping body position during a treatment period.

As further shown in FIG. 15, device 611 comprises a lead 617 including a lead body 618 for chronic subcutaneous implantation (e.g., via tunneling) and extends to a position adjacent a nerve, such as a hypoglossal nerve 605, an ansa cervicalis-related nerve (as one example of nerve 606) and/or phrenic nerve (as one example of nerve 606). The lead 617 may comprise a stimulation electrode to engage the nerve (e.g., 605, 606) for stimulating the nerve to treat a physiologic condition, such as sleep disordered breathing like obstructive sleep apnea, central sleep apnea, multiple-type sleep apneas, etc. The device 633 may comprise circuitry, power element, etc. to support control and operation of both the sensor 635 and the stimulation electrode 612 (via lead 617). In some examples, such control, operation, etc. may be implemented, at least in part, via a control portion (and related functions, portions, elements, engines, parameters, etc.) such as described later in association with at least FIGS. 16A-18.

With regard to the various examples of the present disclosure, in some examples, delivering stimulation to an upper airway patency nerve (e.g., a hypoglossal nerve 605, ansa cervicalis-related nerve as one example of nerve 606, etc.) via the stimulation electrode 612 is to cause contraction of upper airway patency-related muscles, which may cause or maintain opening of the upper airway (608) to prevent and/or treat obstructive sleep apnea. Similarly, such electrical stimulation may be applied to a phrenic nerve (as one example of nerve 606) via the stimulation electrode 612 to cause contraction of the diaphragm as part of preventing or treating at least central sleep apnea. It will be further understood that some example methods may comprise treating both obstructive sleep apnea and central sleep apnea, such as but not limited to, instances of multiple-type sleep apnea in which both types of sleep apnea may be present at least some of the time. In some such instances, separate stimulation leads 617 may be provided or a single stimulation lead 617 may be provided but with a bifurcated distal portion (or trifurcated distal portions) with each separate distal portion extending to a respective one of the hypoglossal nerve 605, ansa cervicalis-related nerve (as one example of nerve 606), and/or the phrenic nerve (as one example of nerve 606).

In some examples, such stimulation electrodes 612, leads 617, and/or the IPG 633 may be arranged to deliver stimulation bilaterally to the hypoglossal nerve 605, ansa cervical is-related nerve (one example of nerve 606), and/or phrenic nerve (one example of nerve 606) including various combinations of simultaneous, alternating, sequential stimulation patterns among the left and right sides, the different nerves, etc.

It will be further understood that in some examples, the stimulation electrode 612, lead 617, and/or IPG 633 (including sensor 635, such as an accelerometer) may be embodied as a microstimulator.

In some such examples, the contraction of the hypoglossal nerve (and/or ansa cervicalis-related nerve) and/or contraction of the phrenic nerve caused by electrical stimulation comprises a suprathreshold stimulation, which is in contrast to a subthreshold stimulation (e.g., mere tone) of such muscles. In one aspect, a suprathreshold intensity level corresponds to a stimulation energy greater than the nerve excitation threshold, such that the suprathreshold stimulation may provide for higher degrees (e.g., maximum, other) upper-airway clearance (i.e., patency) and sleep apnea therapy efficacy.

In some examples, a target intensity level of stimulation energy is selected, determined, implemented, etc. without regard to intentionally establishing a discomfort threshold of the patient (such as in response to such stimulation). Stated differently, in at least some examples, a target intensity level of stimulation may be implemented to provide the desired efficacious therapeutic effect in reducing sleep disordered breathing (SDB) without attempting to adjust or increase the target intensity level according to (or relative to) a discomfort threshold.

In some examples, the treatment period (during which stimulation may be applied at least part of the time) may comprise a period of time beginning with the patient turning on the therapy device and ending with the patient turning off the device. In some examples, the treatment period may comprise a selectable, predetermined start time (e.g., 10 p.m.) and selectable, predetermined stop time (e.g., 6 a.m.). In some examples, the treatment period may comprise a period of time between an auto-detected initiation of sleep and auto-detected awake-from-sleep time. With this in mind, the treatment period corresponds to a period during which a patient is sleeping such that the stimulation of the upper airway patency-related nerve and/or central sleep apnea-related nerve is generally not perceived by the patient and so that the stimulation coincides with the patient behavior (e.g., sleeping) during which the sleep disordered breathing behavior (e.g., central or obstructive sleep apnea) would be expected to occur. In some examples the initiation or termination of the treatment period may be implemented automatically based on sensed sleep state information, which in turn may comprise sleep stage information.

To avoid enabling stimulation prior to the patient falling asleep, in some examples stimulation can be enabled after expiration of a timer started by the patient (to enable therapy with a remote control), or enabled automatically via sleep stage detection. To avoid continuing stimulation after the patient wakes, stimulation can be disabled by the patient using a remote control, or automatically via sleep stage detection. Accordingly, in at least some examples, these periods may be considered to be outside of the treatment period or may be considered as a startup portion and wind down portion, respectively, of a treatment period.

In some examples, stimulation of an upper airway patency-related nerve may be performed via open loop stimulation. In some examples, the open loop stimulation may refer to performing stimulation without use of any sensory feedback of any kind relative to the stimulation.

In some examples, the open loop stimulation may refer to stimulation performed without use of sensory feedback by which timing of the stimulation (e.g., synchronization) would otherwise be determined relative to respiratory information (e.g., respiratory cycles). However, in some such examples, some sensory feedback may be utilized to determine, in general, whether the patient should receive stimulation based on a severity of sleep apnea behavior.

Conversely, in some examples and as previously described in relation to at least several examples, stimulation of an upper airway patency-related nerve may be performed via closed loop stimulation. In some examples, the closed loop stimulation may refer to performing stimulation at least partially based on sensory feedback regarding parameters of the stimulation and/or effects of the stimulation. In some examples, open loop stimulation may be used in response to the confidence factor of the sensory feedback (e.g., sensor signal) falling below a threshold (e.g., 90%, 95%, 99%). When the confidence factor of the sensory feedback rises back above the threshold, closed loop stimulation based on the sensory feedback may be resumed.

In some examples, the closed loop stimulation may refer to stimulation performed via use of sensory feedback by which timing of the stimulation (e.g., synchronization) is determined relative to respiratory information, such as but not limited to respiratory cycle information, which may comprise onset, offset, duration, magnitude, morphology, etc. of various features of the respiratory cycles, including but not limited to the inspiratory phase, expiratory active phase, etc. In some examples, the respiration information excludes (i.e., is without) tracking a respiratory volume and/or respiratory rate. In some examples, stimulation based on such synchronization may be delivered throughout a treatment period or throughout substantially the entire treatment period. In some examples, such stimulation may be delivered just during a portion or portions of a treatment period.

In some examples of "synchronization", synchronization of the stimulation relative to the inspiratory phase may extend to a pre-inspiratory period and/or a post-inspiratory phase. For instance, in some such examples, a beginning of the synchronization may occur at a point in each respiratory cycle which is just prior to an onset of the inspiratory phase. In some examples, this point may be about 200 milliseconds, or 300 milliseconds prior to an onset of the inspiratory phase.

In some examples in which the stimulation is synchronous with at least a portion of the inspiratory phase, the upper airway muscles are contracted via the stimulation to ensure they are open at the time the respiratory drive controlled by the central nervous system initiates an inspiration (inhalation). In some such examples, in combination with the stimulation occurring during the inspiratory phase, example implementation of the above-noted pre-inspiratory stimulation helps to ensure that the upper airway is open before the negative pressure of inspiration within the respiratory system is applied via the diaphragm of the patient's body. In one aspect, this example arrangement may minimize the chance of constriction or collapse of the upper airway, which might otherwise occur if flow of the upper airway flow were too limited prior to the full force of inspiration occurring. In some such examples, the stimulation of the upper airway patency-related nerve may be synchronized to occur with at least a portion of the expiratory period.

With regard to at least the methods of treating sleep apnea as previously described in association with at least FIGS. 1A-14K, at least some such methods may comprise performing the delivery of stimulation to the upper airway patency-related first nerve without synchronizing such stimulation relative to a portion of a respiratory cycle. In some instances, such methods may sometimes be referred to as the previously described open loop stimulation.

In some examples, the term "without synchronizing" may refer to performing the stimulation independently of timing of a respiratory cycle. In some examples, the term "without synchronizing" may refer to performing the stimulation while being aware of respiratory information but without necessarily triggering the initiation of stimulation relative to a specific portion of a respiratory cycle or without causing the stimulation to coincide with a specific portion (e.g., inspiratory phase) of respiratory cycle.

In some examples, in this context the term "without synchronizing" may refer to performing stimulation upon the detection of sleep disordered breathing behavior (e.g., obstructive sleep apnea events) but without necessarily triggering the initiation of stimulation relative to a specific portion of a respiratory cycle or without causing the stimulation to coincide with the inspiratory phase. At least some such examples may be described in Wagner et al., STIMULATION FOR TREATING SLEEP DISORDERED BREATHING, published as US 2018/0117316 on 5/3/2018.

In some examples, while open loop stimulation may be performed continuously without regard to timing of respiratory information (e.g., inspiratory phase, expiratory phase, etc.) such an example method and/or system may still comprise sensing respiration information for diagnostic data and/or to determine whether (and by how much) the continuous stimulation should be adjusted. For instance, via such respiratory sensing, it may be determined that the number of sleep disordered breathing (SDB) events are too numerous (e.g., an elevated AHI) and therefore the intensity (e.g., amplitude, frequency, pulse width, etc.) of the continuous stimulation should be increased or that the SDB events are relative low such that the intensity of the continuous stimulation can be decreased while still providing therapeutic stimulation. It will be understood that via such respiratory sensing, other SDB-related information may be determined which may be used for diagnostic purposes and/or used to determine adjustments to an intensity of stimulation, initiating stimulation, and/or terminating stimulation to treat sleep disordered breathing. It will be further understood that such "continuous" stimulation may be implemented via selectable duty cycles, train of stimulation pulses, selective activation of different combinations of electrodes, etc.

In some examples of open loop stimulation or closed loop stimulation, some sensory feedback may be utilized to determine, in general, whether the patient should receive stimulation based on a severity of sleep apnea behavior. In other words, upon sensing that a certain number of sleep apnea events are occurring, the device may implement stimulation.

Some non-limiting examples of such devices and methods to recognize and detect the various features and patterns associated with respiratory effort and flow limitations include, but are not limited to: Christopherson et al., US 8,938,299, SYSTEM FOR TREATING SLEEP DISORDERED BREATHING, issued January 20, 2015; Christopherson et al., U.S. Patent 5,944,680, titled RESPIRATORY EFFORT DETECTION METHOD AND APPARATUS; and Testerman, U.S. Patent 5,522,862, titled METHOD AND APPARATUS FOR TREATING OBSTRUCTIVE SLEEP APNEA.

Moreover, in some examples various stimulation methods may be applied to treat obstructive sleep apnea, which include but are not limited to: Ni et al., SYSTEM FOR SELECTING A STIMULATION PROTOCOL BASED ON SENSED RESPIRATORY EFFORT, which issued as U.S. 10,583,297 on 3/10/2020; Christopherson et al., US 8938299, SYSTEM FOR TREATING SLEEP DISORDERED BREATHING, issued January 20, 2015; and Wagner et al., STIMULATION FOR TREATING SLEEP DISORDERED BREATHING, published as US 2018/0117316 on 5/3/2018.

In some examples, the example stimulation element(s) 612 shown in FIG. 15 may comprise at least some of substantially the same features and attributes as described in Bonde et al., U.S. 8,340,785, SELF EXPANDING ELECTRODE CUFF, issued on December 25, 2012 and Bonde et al., U.S. 9,227,053, SELF EXPANDING ELECTRODE CUFF, issued on January 5, 2016; Johnson et al., U.S. 8,934,992, NERVE CUFF issued on January 13, 2015; and Rondoni et al., CUFF ELECTRODE, WO 2019/032890 published on February 14, 2019, and filed as U.S. application Serial Number 16/485,954 on August 14, 2019. Moreover, in some examples a stimulation lead 617, which may comprise one example implementation of a stimulation element, may comprise at least some of substantially the same features and attributes as the stimulation lead described in U.S. Patent No. 6,572,543 to Christopherson et al.

In some examples, the stimulation electrode 612 may be delivered transvenously, percutaneously, etc. In some such examples, a transvenous approach may comprise at least some of substantially the same features and attributes as described in Ni et al., Transvenous Method of Treating Sleep Apnea, issued as U.S. 9,889,299 on February 13, 2018. In some such examples, a percutaneous approach may comprise at least some of substantially the same features and attributes as described in Christopherson et al., Percutaneous Access For Systems and Methods Of Treating Sleep Apnea, issued as U.S. 9,486,628 on November 8, 2016.

FIG. 16A is a block diagram schematically representing example method 700. In some examples, method 700 may be implemented via at least some of the devices, sensors, sensing elements, etc. as previously described in association with FIGS. 1A-15. In some examples, the example method 700 may be at least partially implemented within, and/or via, control portion 900 in FIG. 17A, control portion 920 in FIG. 17B, or interface 940 in FIG. 18.

As shown at 710 in FIG. 16A, the example method 700 comprises sensing acceleration signal(s) from a sensor(s) implanted within a patient's body in a position, such as in the chest region, to detect respiration information. In some examples, just a single sensing element (e.g., 122A in FIG. 3) may be used to provide just a single sensed acceleration signal or in some examples, multiple sensing elements may be used to provide separate multiple sensed acceleration signals. The multiple sensing elements may be separate from, and independent of, each other, or may be co-located as part of a single device, such as a three-axis accelerometer.

As further shown at 714, filtering is applied separately to the sensed signal(s) (710) to produce a respective separate inclination angle signal (721X, 721Y, 721Z) for each corresponding acceleration signal (e.g., X-axis, Y-axis, Z-axis). It will be understood that if just one single-axis sensing element is employed, then just one inclination angle signal will be present at 720. As previously described through various examples, the inclination angle signal represents the physiologic phenomenon of the patient's breathing with a value and/or shape of the inclination angle signal varying through the different phases of a respiratory cycle (e.g., inspiratory phase, expiratory active phase, expiratory pause phase) as the patient breathes. It will be further noted that while some examples may comprise tracking inclination angle signal for multiple axes (X, Y, Z), some example methods may focus on an axis which is closest to being generally perpendicular to the gravity vector.

In some examples, in addition to applying filtering (at 714) as described above to produce a respective separate inclination angle signal (721X, 721Y, 721Z) for each corresponding acceleration signal (e.g., X-axis, Y-axis, Z-axis), the filtering may further comprise subtracting (e.g., filtering, excluding) noise from the signal to increase the signal-to-noise ratio for the respiratory features of interest. In some examples, such noise filtering may be implemented as described later in association with noise model parameter 870 in FIG. 16E. It will be understood that in some examples, such noise filtering may be applied in other ways and/or at other times within the example method (and/or arrangement) in FIG. 16A.

As further shown at 740 in FIG. 16A, method 700 comprises performing a feature extraction on a signal-by-signal basis (741X, 741Y, 741Z) to identify within each inclination angle signal (721X, 721Y, 721Z) features indicative of respiration (and/or other features pertinent to respiratory detection, patient health, etc.), such as the expiratory phase midpoints and/or the inspiratory phase midpoints as described with reference to at least FIGS. 5A and 5B. In some examples, a non-midpoint of an inspiratory phase and/or an expiratory phase may be used as described in association with at least FIGS. 5C and 5D. As shown at 750, in some examples the method identifies at least respiratory phase information including (but not limited to) the features of an inspiratory phase 752, an expiratory active phase 754, and an expiratory pause phase 756. It will be understood that each phase 752, 754, 756 may comprise a start (i.e., onset), an end (e.g., offset), duration, magnitude, and/or both a "start and end" of each respective phase. In some instances, a particular feature may sometimes be referred to as a fiducial or similar term, such as a start of a phase (e.g., inspiration) comprising a fiducial or a midpoint of a phase (e.g., inspiration) comprising a fiducial.

As shown at 730, a confidence factor may be applied to each of the feature extraction elements (741X, 741Y, 741Z), such as an X-axis confidence factor 731X, Y-axis confidence factor 731Y, and Z-axis confidence factor 731Z. At least some aspects of applying a confidence factor are described later in association with at least FIG. 16B. In some examples, the feature extraction elements (741X, 741Y, 741Z) having the highest confidence factor are used at 745.

In other examples, upon performing feature extraction (741X, 741Y, 741Z) of respiratory phase information to each inclination angle signal, the resulting extracted feature signals are combined (e.g., fused together) at 745 to produce (i.e., determine) a composite sensed respiratory signal including respiratory phase information (750) including inspiratory phase 752, expiratory active phase 754, and expiratory pause phase 754. In some examples, the different extracted feature signals may be combined (e.g., fused) as an average of the respective features, a median of the respective features, or weighting (linear or non-linear) according to a confidence factor (e.g., 731X, 731Y, 731Z). At least some aspects of the confidence factor(s) are described later in association with at least FIG. 16B. In some such examples, the composite sensed respiratory signal may correspond to the virtual vector as previously described in association with at least FIG. 8 and throughout various examples of the present disclosure.

As further shown in FIG. 16A, from the determined respiratory phases (752, 754, 754), additional respiratory parameters 760 may be determined. For example, an (overall) expiratory phase may comprise a sum or combination of the expiratory active phase (754) and the expiratory pause phase (756). In addition, a respiratory period may be determined from a sum of duration of the inspiratory phase 752 and a duration of the (overall) expiratory phase, including both the active and pause phases 754, 756. Meanwhile, the respiratory rate (RR) may be computed as 1/respiratory period. Additional parameters may comprise a computed I/E ratio, such as inspiratory phase duration (Ti in FIG. 4) divided by an expiratory phase duration ($T_{EA}$ plus $T_{EP}$ in FIG. 4).

In some examples, assuming a given body position or posture and excluding translational motion along the axes (e.g., X, Y, Z), some additional parameters may be determined from the extracted features (including respiratory phase information at 750) with such additional parameters comprising: an approximation of a tidal volume as being proportional to acceleration; an approximation of respiratory flow as being proportional to a derivative of the acceleration signal with respect to time; and/or an approximation of minute ventilation as being proportional to a result of a multiplication of the computed volume and the computed respiratory rate (described above).

In some examples, determinations relating to feature extraction (740 in FIG. 16A) may further comprise the following parameters. For instance, in some examples of feature extraction, a signal midpoint may be determined as previously described with reference to FIGS. 5A and 5B. In some examples of feature extraction, a signal midpoint crossing may be determined, which comprises a sample at which the signal midpoint is crossed. In some examples, the signal midpoint crossing may involve hysteresis with a hysteresis threshold being determined by a fixed threshold, a fraction of recent "n" peak-to-peak values, a fraction of signal root-mean-square (RMS) value, and/or a dynamic threshold with linear decay or exponential decay.

With further reference to FIG. 16A, in some examples determining respiration information (via sensing acceleration signals to detect rotational movements of the ribcage during breathing), the example method 700 may utilize default respiratory phase values as shown at 790 instead of using the sensed acceleration signals 710. For instance, in cases in which the sensed acceleration signal quality is poor (i.e., inadequate), the current respiratory phases of the patient may not be known from the current sensed acceleration signals or recent sensed acceleration signals. In some examples, the default respiratory phase values (790) are assigned a confidence level or factor 791, which may have a low value to ensure that extracted features (741X, 741Y, 741Z) are used when the sensed acceleration signal quality is adequate. Accordingly, when the sensed acceleration signal is of sufficient quality as determined by the signal-to-noise ratio of the signal, then method 700 may ignore the default respiratory phase values at 790. The signal-to-noise ratio may be determined by a comparison with a typical signal morphology, a comparison with a typical signal frequency content, or by other means.

With further reference to the default respiratory phase values portion 790 in FIG. 16A, in some examples the default respiratory phase values (790) may be determined using at least one of the following: (1) mean respiratory phase time values of the overall human population; (2) the patient's historical or recent mean/median respiratory phase and/or phase time values; and (3) intentionally applying a longer respiratory rate or a shorter respiratory rate to decrease the chance that an appreciable number of consecutive stimulation "off" times may align with inspiration.

Accordingly, via the default respiratory phase values, some example methods may comprise substituting, upon the sensor obtaining an inadequate signal, stored respiratory information comprising historical respiration information for at least one of: the patient's respiratory cycle information; and multiple-patient respiratory cycle information. In some such examples, the patient's respiratory cycle information comprises a respiratory period, and an example method comprises: creating a modified respiratory period by adding a random time value to the respiratory period of the patient's respiratory cycle information; and implementing the substituting of the stored respiratory information using the modified respiratory period. In some examples, the random time value may comprise about 0 to about 1 second. In some examples, the random time value may comprise other time periods. In some examples, adding the random time value may cause a result similar to that noted above (in regard to the default respiratory phase values) by which the example method may intentionally apply a longer respiratory rate or a shorter respiratory rate to decrease the chance that an appreciable number of consecutive stimulation "off" times may align with inspiration.

In some examples, the method may comprise substituting, upon the sensor obtaining an inadequate signal, stored respiratory information comprising respiratory cycle information including at least one of: a first respiratory rate substantially faster than the patient's average respiratory rate; and a second respiratory rate substantially slower than the patient's average respiratory rate. In some such examples, the terms substantially faster and/or substantially slower may correspond to a difference on the order of 5 percent difference, 10 percent difference, and the like.

FIG. 16B is a block diagram schematically representing an example confidence factor portion 800, which may be employed at 730 in example method 700 and/or as part of (or via) control portion 900 in FIG. 17A. It will be understood that all or just some of the factors (e.g., different combinations or a single factor) in confidence factor portion 800 may be applied at 730 in method 700 in FIG. 16A. In some examples, a confidence factor may be implemented as an estimated probability of correctness.

As shown in FIG. 16B, in some examples confidence factor portion 800 comprises a first factor portion 810 comprising a signal-to-noise ratio parameter 812, a threshold parameter 814, and a recent history parameter 816. Accordingly, in some examples, via signal-to-noise ratio information (parameter 812), a confidence level may be determined for each extracted feature (at 740 in FIG. 16A) and/or for each inclination angle signal (at 720 in FIG. 16A). In some examples, at 814 method 700 comprises the confidence comprising an amount by which a value (e.g., of a feature, of the inclination signal, etc.) exceeds a threshold. Stated differently, if a value of the inclination signal such as for a particular axis (e.g., Y-axis in FIG. 3) exceeds a threshold by a significant amount, then the method can apply a high value confidence factor to the Y-axis feature extraction (741Y in FIG. 16A) such that determination of the respiratory phase information (750 in FIG. 16A) may depend primarily on the Y-axis inclination signal (721Y in FIG. 16A) as compared to other axes (e.g., X or Z) inclination signals, if present. In some examples, the confidence factor may be applied per recent history parameter 816 according to a difference between a current value of an extracted feature and a mean value of "n" recent extracted features. In some examples, each of the confidence parameters in first factor portion 810 may be applied quantitatively according to a look-up table, multiplication factor (e.g., 1.5, 2x, etc.), and the like.

In some examples, confidence factor portion 800 may comprise a second factor portion 820 by which confidence in a value of a particular extracted feature (741X, 741Y, 741Z) may be increased or decreased based on posture (822) at the time of sensing, heart rate (824), and/or sleep stage (826). As further shown in third factor portion 830 of FIG. 16B, such confidence factors in second factor portion 820 may be weighted and/or calibrated according to particular patient-based factors, such as patient preferences (e.g., feedback) 832, clinician input 834, and/or other information such sleep study information. Further parameters which may comprise part of second confidence factor portion 820 may include sensed body temperature, time of day, etc.

In some examples, the various parameters, etc. of the respective first, second, and third portions of confidence factor portion 800 may be used together in different combinations and/or organized in different groupings (or no groupings) than shown in FIG. 16B.

FIG. 16C is a block diagram schematically representing an example feature extraction portion 850, which may comprise functions, settings, etc. which may act as part of the implementation of the feature extraction at 740 in method 700 of FIG. 16A. As shown via parameter 852 at 850 in FIG. 16C, in some examples a threshold factor may be applied by a user or clinician to adjust thresholds used in performing feature extraction of the inspiratory phase 752 (e.g., inhalation threshold), of the expiratory active phase 754 (e.g., exhalation threshold), and/or of the expiratory pause phase 756 (e.g., exhalation threshold). As shown via parameter 854 at 850 in FIG. 16C, in some examples a sensitivity factor may be applied by a user or clinician to adjust thresholds used in performing feature extraction of the inspiratory phase 752 (e.g., inhalation sensitivity), of the expiratory active phase 754 (e.g., exhalation sensitivity), and/or of the expiratory pause phase 756 (e.g., exhalation sensitivity).

In some examples, in determining the respiratory phase information (790) example method 700 also may comprise predicting an inspiratory phase (e.g., 752 in FIG. 16A), as shown at 860 in FIG. 16D. The prediction of the inspiratory phase may be used to increase a likelihood of implementing actions (e.g., start of stimulation, etc.) which are to be synchronized with a start of the inspiratory phase 752. Stated differently, predicting the inspiratory phase 752 as at 860 in FIG. 16D may decrease a chance that detection of a start of the inspiratory phase might be missed. Moreover, in some example methods and/or example devices, electrical stimulation of a nerve (e.g., hypoglossal nerve, ansa cervicalis-related nerve) may be initiated prior to a start of inspiration to ensure that the upper airway is open prior to the pressure applied on the upper airway once the actual inspiratory phase commences. In addition, starting electrical stimulation prior to the actual inspiratory phase also may provide some assurance in cases in which prediction of the inspiratory phase may be incorrect or may experience an insufficient signal-to-noise ratio. In some such examples, example methods and/or devices may initiate the stimulation a predetermined period of time prior to an onset of the inspiratory phase. In some examples, the predetermined period of time has a duration less than a duration of the expiratory pause according to an average duration of an expiratory pause phase, according to a duration of the preceding expiratory pause phase, etc. In some examples, the predetermined period of time may comprise an absolute amount of time (e.g., start 0.5 seconds) and in some examples, the predetermined period of time may comprise a relative amount of time, such as 10% of the preceding respiratory period. As mentioned in association with other examples regarding synchronization, in some examples the predetermined period of time may be about 200 milliseconds or 300 milliseconds.

In some examples, the inspiratory phase prediction function (860) in FIG. 16D may comprise predicting a start of the inspiratory phase via timing based on: (1) an expiratory active phase 754 of the most recent (e.g., immediately preceding) respiratory cycle; (2) an expiratory pause phase 756 of the most recent (e.g., immediately preceding) respiratory cycle; and/or (3) an inspiratory phase of one or more previous respiratory cycles and/or the respiratory rate of one or more previous respiratory cycles. In some examples, in determining the timing (of the inspiratory phase and/or respiratory rate of previous respiratory cycles), the method may utilize a mean value, a median value, linear extrapolation, and/or non-linear extrapolation of the respective inspiratory phase or respiratory rate.

With further reference to inspiratory phase prediction 860 in FIG. 16D, in some examples, determining the timing (of the inspiratory phase and/or respiratory rate of previous respiratory cycles), the use of values from previous respiratory cycles may also enhance an accuracy of feature extraction (740 in FIG. 16A). For instance, accuracy of timing peak detection may be enhanced by using data before and after the peak. In another instance, using values from previous respiratory cycles may make an example method (of detecting respiration) less susceptible to a noisy signal during a particular respiratory cycle, patient limb movements, bed partner movements, etc.

In some examples, a method may increase accuracy of determining respiration from a sensed acceleration signal (of rotational movement at a portion of a chest wall) by removing noise from the sensed signal according to a noise model, which is shown in association with at least noise model parameter 870 in FIG. 16E.

In some such examples, the method comprises constructing the noise model from identifying characteristics (e.g., signal morphology, frequency content, etc.) within the sensed signal which are caused by and/or associated with conditions, phenomenon, etc. other than respiration-related behavior of the patient (and/or cardiac-related behavior, etc.) and which are considered noise relative to the signal of interest regarding patient respiration. In just one example, one source of noise (which may form at least part of a noise model) may comprise movement, behavior, etc. from another person (i.e., partner) sleeping in the same bed, which may be picked up by the sensed signal for the patient. In some instances, such motion may sometimes be referred to as non-patient-physiologic motion. Other sources of noise, which form at least part of a noise model, may comprise additional/other non-patient-physiologic motion, such as but not limited to motion of a vehicle in which the patient is present such as when the patient is traveling a car, airplane, spaceship, etc. Other types of non-patient-physiologic motion which may be considered as noise (and which form at least part of a noise model) may comprise movement of a patient support surface, such as a hammock, swings, etc. Another type of noise, which may form at least part of the noise model, may comprise a physical position of the patient such as being in a very tall building in motion due to wind, a location experiencing vibration or movement such that the motion of the patient may affect the sensed acceleration signal and otherwise hinder accurate determination of respiration information per the type of rotational sensing in the examples of the present disclosure.

By constructing a noise model from these non-patient characteristics, and subtracting the noise model from the sensed acceleration signal of the patient, a more accurate sensed respiration signal may be determined. In some instances, the subtraction may be performed by filtering the noise and/or by excluding sensor element signals including such noise.

In some examples, such noise may be filtered or excluded from the sensed acceleration signals (of rotational movement of a respiratory body portion, such as a chest wall) without use of a formal noise model.

In some examples, at least some of the features and attributes of use of a noise model, which may increase a signal-to-noise ratio of the signal of interest (respiration information), may be implemented at least partially within or via filtering 714 in method 700 as shown in FIG. 16A.

In some examples, the prediction of the inspiratory phase (e.g., 860 in FIG. 16D) also may be performed according to cross-referencing (e.g., similarity) the inspiratory phase of a previous respiratory cycle relative to stored reference morphology of the inspiratory phase.

FIG. 16F is a block diagram schematically representing another example method 770. In some examples, method 770 may be implemented via at least some of the devices, sensors, sensing elements, etc. as previously described in association with FIGS. 1A-15. In some examples, the example method 770 may be at least partially implemented within, and/or via, control portion 900 in FIG. 17A, control portion 920 in FIG. 17B, or interface 940 in FIG. 18.

As shown at 710 in FIG. 16F, the example method 770 comprises sensing acceleration signal(s) from a sensor(s) implanted within a patient's body in a position, such as in the chest region, to detect respiration information. In some examples, just a single sensing element (e.g., 122A in FIG. 3) may be used to provide just a single sensed acceleration signal or in some examples, multiple sensing elements may be used to provide separate multiple sensed acceleration signals. The multiple sensing elements may be separate from, and independent of, each other, or may be co-located as part of a single device, such as a three-axis accelerometer.

The sensed acceleration signals 710 may comprise measured gravity vectors $A_i = \langle Ax_i, Ay_i, Az_i \rangle$, (corresponding to the X-axis, the Y-axis, and the Z-axis as illustrated, for example, in FIG. 8), and may be stored in a buffer $\{A_i\}_{i=1..N}$. In some examples, the quality assessment block 780 determines if the signals in the buffer are adequate, i.e., the signals only show a small variation consistent with breathing. For example, the quality assessment block 780 may calculate the total variance $V$:

$$V = \frac{1}{N} \sum_{i=1}^{N} [(Ax_i - \overline{Ax})^2 + (Ay_i - \overline{Ay})^2 + (Az_i - \overline{Az})^2]$$

where $\overline{Ax}$ is the average of the $Ax_i$, $\overline{Ay}$ is the average of the $Ay_i$, and $\overline{Az}$ is the average of the $Az_i$. If $V$ falls below some threshold, then the signals are considered to be adequate. The threshold may be a parameter that is programmed into the device, or the threshold may be determined adaptively from a patient's signals.

The adequate signals are passed through filters 772 to give both an average gravity vector $G$ and bandpass filtered signals $F_i = \langle Fx_i, Fy_i, Fz_i \rangle$. The bandpass filter removes both the low frequency (slowly varying) components that relate to patient posture and the high frequency components that contain unwanted signals (e.g., those relating to cardiac motion) and noise. The bandpass filter may operate in one of several modes, for example:

- The filter takes all raw signals as input, but output from the filter is only taken when the raw signal is considered to be adequate.
- The filter only takes adequate signals as input, and is reset when inadequate signals are received.
- The adequate signals are fed into a buffer and, when the buffer is full, the filter operates on the buffer to produce output.

The filtered signals may also be buffered. It is useful to have contiguous signals, so when inadequate signals are detected, the buffer may be emptied and only full buffers may be passed on to the later stages of processing.

The next stage of signal processing involves extracting a virtual vector 776 (e.g., one, scalar breathing component from the vector of filtered signals). In the world frame of reference the (reaction to the) gravity vector, $G_w$ (e.g., G in FIG. 2), is fixed and the sensor mostly rotates about some axis $R_w$ (e.g., B1 in FIG. 2) due to the patient's breathing. The sensor measures signals in its frame of reference. The measured gravity vector $G$ rotates around an axis $R$, which is expected to only vary slowly, at least when the patient is lying still. As a result, the variation in $G$ due to breathing mostly occurs along an axis $B$ orthogonal to $R$ and $G$, i.e., parallel to the vector cross product $R \times G$.

The breathing axis can be determined from the filtered signals using various methods. In one example as described with reference to FIG. 8, Principal Component Analysis is performed on the filtered signals to determine the virtual vector 776.

As further shown at 778 in FIG. 16F, method 770 comprises performing a feature extraction on the virtual vector 776 to identify features indicative of respiration (and/or other features pertinent to respiratory detection, patient health, etc.), such as the expiratory phase midpoints and/or the inspiratory phase midpoints as described with reference to at least FIGS. 5A and 5B. In some examples, a non-midpoint of an inspiratory phase and/or an expiratory phase may be used as described in association with at least FIGS. 5C and 5D.

As shown at 781, a confidence factor may be applied to each of the feature extraction elements 778. The confidence is reduced if the variance measured in the quality assessment is too large (indicating signal corruption, e.g., by patient motion) or too small (indicating reduced signal-to-noise ratio).

If PCA is used to estimate the breathing axis, the second singular value can also be computed, and the confidence is reduced if the second singular value is not small compared to the first singular value. That is, PCA takes a window of X,Y,Z accelerometer data and decomposes it into three components, each of which comprises a virtual vector, a signal, and a "singular value" that measures the strength of the signal along the virtual vector. The breathing signal is expected to correspond to the first component, which has the largest singular value, i.e., the strongest signal. If the first singular value is much larger than the second singular value (e.g., by at least a factor of 2), the confidence that the breathing signal has been correctly identified is higher. If the second singular value is close to the first singular value (e.g., within a factor of 1.5), the two signals are comparable in strength and the confidence that the breathing signal has been correctly identified is lower.

If least squares fitting is used to estimate the frequency and/or phase of the breathing signal, the confidence is reduced if the residual for this fit is large. That is, the residual is the difference between the original breathing signal and the fitted signal. The quality of the fit can be measured as the variance of the residual divided by the variance of the original signal. If this number is low (i.e., the residual is small compared to the original signal) then the confidence in the fit is higher.

If a locus $(b(t), b1(t))$ is used to estimate frequency and phase, the confidence is reduced if the estimates are inconsistent over several t values.

The motion processing chain may predict the start of inspiration for more than one future respiratory cycle, e.g., for two or three future respiratory cycles. Each prediction can have an associated confidence. These predictions can be replaced by later predictions if the confidence of the prediction has improved. If the confidence of the predicted start of inspiration is too low, the stimulation can be applied according to some default pattern rather than the predicted time for the start of inspiration, e.g., an aperiodic sequence with an average frequency similar to typical breathing frequencies.

The breathing phase and, optionally, also the frequency, may be determined to enable prediction of the start of the next inspiration. The first step is to fix the polarity of the breathing signal. Note that the methods above for determining the breathing axis $B$ could return $B$ or $-B$, and similarly $b$ or $-b$ for the breathing signal. If the breathing signal was a pure sinusoid, there is no way to distinguish between $-b$ and $b$ with a phase shift of 180°. This would result in confusion between inspiration and expiration. However, real breathing signals have an asymmetry. As illustrated in FIG. 4, the expiration phase tends to end with a pause, giving a short plateau before the start of inspiration.

As shown at 750 and previously described with reference to FIG. 16A, in some examples the method 770 identifies at least respiratory phase information including (but not limited to) the features of an inspiratory phase 752, an expiratory active phase 754, and an expiratory pause phase 756. As further shown in FIG. 16A, from the determined respiratory phases (752, 754, 754), additional respiratory parameters 760 may be determined as previously described. In some examples, determining respiration information (via sensing acceleration signals to detect rotational movements of the ribcage during breathing), the example method 770 may utilize default respiratory phase values as shown at 790 instead of using the sensed acceleration signals 710 as previously described.

The respiratory information relates to breathing frequency and phase, which can be used to predict the time of the next start of inspiration, when simulation may be applied. This may combine short-term predictions, e.g., based on the shape of $b(t)$ over a short time window, and long-term predictions, e.g., based on the average frequency and phase of $b(t)$ over the last few breaths. The short-term predictions and the long-term predictions may be combined.

In some examples, inspiration may be detected on-the-fly, where the predicted start of inspiration is mostly based on the short-term behavior of $b(t)$. The longer-term behavior is only used for sanity-checking, e.g., the next predicted start time should be at least some minimum interval after the previous stimulation period. On-the-fly detection is advantageous for times when the period of a patient's breath varies significantly from breath to breath. A drawback of on-the-fly detection includes a greatest delay between the start of inspiration and the stimulation, because the predicted start time is likely to be within the window of past $b(t)$ data.

In other examples, inspiration may be detected based on several breaths, where the predicted start of inspiration is mostly based on the long-term behavior of $b(t)$, i.e., based on estimates of frequency and phase. Inspiration prediction based on long-term behavior is advantageous for robust operation without large errors. A drawback of inspiration prediction based on long-term behavior includes timing errors, which may occur when the period of a patient's breath varies significantly from breath to breath.

In yet other examples, inspiration may be detected based on variable trigger sensitivity, where the predicted start of inspiration is mostly based on the short-term behavior of $b(t)$, but the prediction is biased to be close to the start time predicted from the long-term behavior. This is a compromise between the on-the-fly detection of inspiration and the inspiration prediction based on the long-term behavior methods above. In some examples, variable trigger sensitivity may learn the breathing waveform from previous breaths and/or use a Kalman filter. The breathing period and inspiration duration may be estimated from several historical breaths, using zero crossings. These may be used to predict the next start of inspiration from each zero crossing that occurs during expiration.

In other examples, variable trigger sensitivity may include fitting sine waves with first and second harmonics. That is, the breathing frequency, $\omega$, may be calculated from the long-term behavior of $b(t)$. The sine wave coefficients may be determined from a single breathing cycle running roughly from expiration to expiration to determine the breathing phase. The breathing frequency and phase may be combined to determine the start of the next inspiration phase, a fraction of a breath ahead in time.

In other examples, variable trigger sensitivity may include peak detection and fitting quadratics. That is, the breathing frequency, $\omega$, and inspiration duration may be calculated from the peaks seen during several historical breaths. These are used to predict the next start of inspiration from each inspiration peak.

As well as predicting the time for the start of inspiration, the times for other breathing phases can also be predicted, e.g., the end of inspiration. For prediction methods based on long-term behavior of $b(t)$, this just involves applying different phase offsets. For short-term prediction, the method can differ with the phase, e.g., one pattern-matching method can be used for the start of inspiration and a different pattern-matching method can be used for the end of inspiration.

FIG. 17A is a block diagram schematically representing an example control portion 900. In some examples, control portion 900 provides one example implementation of a control portion forming a part of, implementing, and/or generally managing sensors, sensing element, respiration determination elements, stimulation elements, power/control elements (e.g., pulse generator), elements, devices, user interfaces, instructions, information, engines, elements, functions, actions, and/or methods, as described throughout examples of the present disclosure in association with FIGS. 1A-16E and 17B-20.

In some examples, control portion 900 includes a controller 902 and a memory 910. In general terms, controller 902 of control portion 900 comprises at least one processor 904 and associated memories. The controller 902 is electrically couplable to, and in communication with, memory 910 to generate control signals to direct operation of at least some of the sensors, sensing element, respiration determination elements, stimulation elements, power/control elements (e.g., pulse generators), devices, user interfaces, instructions, information, engines, elements, functions, actions, and/or methods, as described throughout examples of the present disclosure. In some examples, these generated control signals include, but are not limited to, employing instructions 911 and/or information 912 stored in memory 910 to at least determining respiration information of a patient. Such determination of respiration information may comprise part of directing and managing treatment of sleep disordered breathing such as obstructive sleep apnea, hypopnea, and/or central sleep apnea. In some instances, the controller 902 or control portion 900 may sometimes be referred to as being programmed to perform the above-identified actions, functions, etc. such that the controller 902, control portion 900 and any associated processors may sometimes be referred to as being a special purpose computer, control portion, controller, or processor. In some examples, at least some of the stored instructions 911 are implemented as, or may be referred to as, a care engine, a sensing engine, a respiration determination engine, a monitoring engine, and/or a treatment engine. In some examples, at least some of the stored instructions 911 and/or information 912 may form at least part of, and/or, may be referred to as a care engine, sensing engine, respiration determination engine, monitoring engine, and/or treatment engine.

In response to or based upon commands received via a user interface (e.g., user interface 940 in FIG. 18) and/or via machine readable instructions, controller 902 generates control signals as described above in accordance with at least some of the examples of the present disclosure. In some examples, controller 902 is embodied in a general purpose computing device while in some examples, controller 902 is incorporated into or associated with at least some of the sensors, sensing element, respiration determination elements, stimulation elements, power/control elements (e.g., pulse generators), devices, user interfaces, instructions, information, engines, functions, actions, and/or method, etc. as described throughout examples of the present disclosure.

For purposes of this application, in reference to the controller 902, the term "processor" shall mean a presently developed or future developed processor (or processing resources) that executes machine readable instructions contained in a memory. In some examples, execution of the machine readable instructions, such as those provided via memory 910 of control portion 900 cause the processor to perform the above-identified actions, such as operating controller 902 to implement the sensing, monitoring, determining respiration information, stimulation, treatment, etc. as generally described in (or consistent with) at least some examples of the present disclosure. The machine readable instructions may be loaded in a random access memory (RAM) for execution by the processor from their stored location in a read only memory (ROM), a mass storage device, or some other persistent storage (e.g., non-transitory tangible medium or nonvolatile tangible medium), as represented by memory 910. In some examples, the machine readable instructions may comprise a sequence of instructions, a processor-executable machine learning model, or the like. In some examples, memory 910 comprises a computer readable tangible medium providing nonvolatile storage of the machine readable instructions executable by a process of controller 902. In some examples, the computer readable tangible medium may sometimes be referred to as, and/or comprise at least a portion of, a computer program product. In other examples, hard wired circuitry may be used in place of or in combination with machine readable instructions to implement the functions described. For example, controller 902 may be embodied as part of at least one application-specific integrated circuit (ASIC), at least one field-programmable gate array (FPGA), and/or the like. In at least some examples, the controller 902 is not limited to any specific combination of hardware circuitry and machine readable instructions, nor limited to any particular source for the machine readable instructions executed by the controller 902.

In some examples, control portion 900 may be entirely implemented within or by a stand-alone device.

In some examples, the control portion 900 may be partially implemented in one of the sensors, sensing element, respiration determination elements, monitoring devices, stimulation devices, apnea treatment devices (or portions thereof), etc. and partially implemented in a computing resource separate from, and independent of, the apnea treatment devices (or portions thereof) but in communication with the apnea treatment devices (or portions thereof). For instance, in some examples control portion 900 may be implemented via a server accessible via the cloud and/or other network pathways. In some examples, the control portion 900 may be distributed or apportioned among multiple devices or resources such as among a server, an apnea treatment device (or portion thereof), and/or a user interface.

In some examples, control portion 900 includes, and/or is in communication with, a user interface 940 as shown in FIG. 18.

Figure 17B is a diagram schematically illustrating at least some example arrangements of a control portion 920 by which the control portion 900 (FIG. 17A) can be implemented, according to one example of the present disclosure. In some examples, control portion 920 is entirely implemented within or by an IPG assembly 925, which has at least some of substantially the same features and attributes as a pulse generator (e.g., power/control element) as previously described throughout the present disclosure. In some examples, control portion 920 is entirely implemented within or by a remote control 930 (e.g., a programmer) external to the patient's body, such as a patient control 932 and/or a physician control 934. In some examples, the control portion 900 is partially implemented in the IPG assembly 925 and partially implemented in the remote control 930 (at least one of patient control 932 and physician control 934).

FIG. 18 is a block diagram schematically representing user interface 940, according to one example of the present disclosure. In some examples, user interface 940 forms part or and/or is accessible via a device external to the patient and by which the therapy system may be at least partially controlled and/or monitored. The external device which hosts user interface 940 may be a patient remote (e.g., 932 in FIG. 17B), a physician remote (e.g., 934 in FIG. 17B) and/or a clinician portal. In some examples, user interface 940 comprises a user interface or other display that provides for the simultaneous display, activation, and/or operation of at least some of the sensors, sensing element, respiration determination elements, stimulation elements, power/control elements (e.g., pulse generators), devices, user interfaces, instructions, information, engines, functions, actions, and/or method, etc., as described in association with FIGS. 1A-20. In some examples, at least some portions or aspects of the user interface 940 are provided via a graphical user interface (GUI), and may comprise a display 944 and input 942.

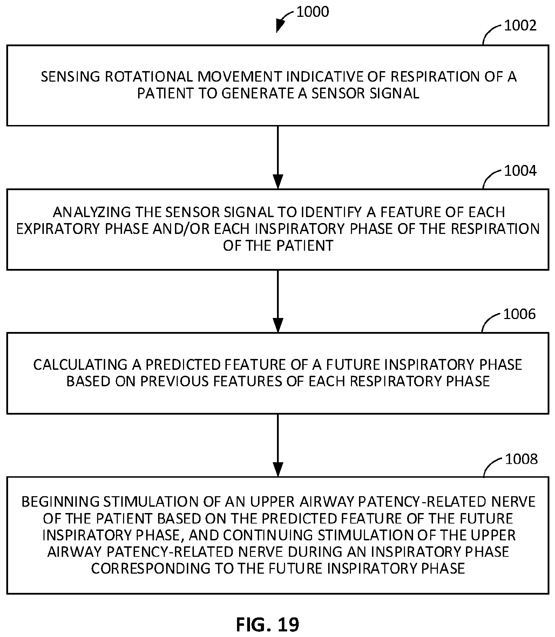

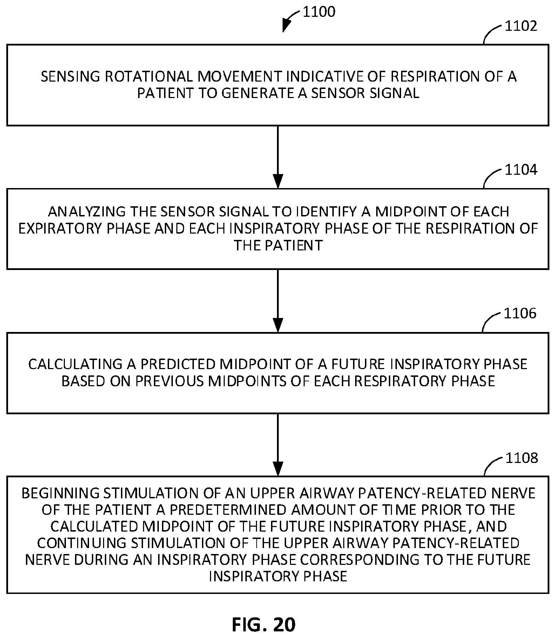

FIGS. 19 and 20 are flow diagrams schematically representing various example methods. In some examples, the various methods in FIGS. 19 and 20 may be implemented via at least some of the sensors, sensing element, respiration determination elements, stimulation elements, power/control elements (e.g., pulse generators), devices, user interfaces, instructions, information, engines, functions, actions, and/or methods, as previously described in association with FIGS. 1A-18. In some examples, the various methods in FIGS. 19 and 20 may be implemented via elements other than those previously described in association with FIGS. 1A-18.

FIG. 19 is a flow diagram illustrating one example of a method 1000 of treating sleep disordered breathing. At 1002, method 1000 includes sensing rotational movement indicative of respiration of a patient to generate a sensor signal. At 1004, method 1000 includes analyzing the sensor signal to identify a feature of each expiratory phase and/or each inspiratory phase of the respiration of the patient. At 1006, method 1000 includes calculating a predicted feature of a future inspiratory phase based on previous features of each respiratory phase. At 1008, method 1000 includes beginning stimulation of an upper airway patency-related nerve of the patient based on the predicted feature of the future inspiratory phase, and continuing stimulation of the upper airway patency-related nerve during an inspiratory phase corresponding to the future inspiratory phase.

FIG. 20 is a flow diagram illustrating another example of a method 1100 of treating sleep disordered breathing. At 1102, method 1100 includes sensing rotational movement indicative of respiration of a patient to generate a sensor signal (e.g., as described with reference to at least FIGS. 1A-5B). At 1104, method 1100 includes analyzing the sensor signal to identify a midpoint of each expiratory phase and each inspiratory phase of the respiration of the patient (e.g., as indicated at 152 and 153 in FIGS. 5A and 5B). At 1106, method 1100 includes calculating a predicted midpoint of a future inspiratory phase based on previous midpoints of each respiratory phase (e.g., as indicated at 156 in FIGS. 5A and 5B). At 1108, method 1100 includes beginning stimulation of an upper airway patency-related nerve of the patient a predetermined amount of time relative to the calculated midpoint of the future inspiratory phase, and continuing stimulation of the upper airway patency-related nerve during an inspiratory phase corresponding to the future inspiratory phase (e.g., as indicated at 157 in FIGS. 5A and 5B).

Although specific examples have been illustrated and described herein, a variety of alternate and/or equivalent implementations may be substituted for the specific examples shown and described without departing from the scope of the present disclosure. The invention is defined by the appended claims.

**Claims**

1. A device comprising:

    a sensor securable to a patient to provide a sensor signal, the sensor to sense changes in acceleration indicative of respiration of the patient; and
    a control portion configured to:

        determine a midpoint of each respiratory phase of the patient based on the sensor signal;
        identify each expiratory phase or each inspiratory phase of the patient based on the sensor signal; and
        predict a midpoint of a future inspiratory phase based on previous midpoints of each respiratory phase.

2. The device of claim 1, wherein the control portion is configured to identify whether the slope of each expiratory phase is positive or negative.

3. The device of claim 2, wherein the control potion is configured to:

    determine the median of data points of the sensor signal between a current midpoint and at least two previous midpoints;
    identify the slope of the expiratory phase as negative in response to the median being less than 0; and
    identify the slope of the expiratory phase as positive in response to the median being greater than 0.

4. The device of claim 1, wherein the control portion is configured to identify whether the slope of each inspiratory phase is positive or negative.

5. The device of claim 4, wherein the control potion is configured to:

    determine the median of data points of the sensor signal between a current midpoint and at least two previous midpoints;
    identify the slope of the inspiratory phase as positive in response to the median being less than 0; and
    identify the slope of the inspiratory phase as negative in response to the median being greater than 0.

6. The device of claim 1, wherein the sensor comprises an accelerometer to sense rotational movement.

7. The device of claim 6, wherein the accelerometer comprises a three axis accelerometer.

8. The device of claim 7, wherein the three axis accelerometer provides three respective sensor signals, and wherein the control portion is configured to:

    determine a confidence factor associated with each of the three respective sensor signals;
    identify which respective sensor signal exhibits the greatest confidence factor; and
    determine the midpoint of each respiratory phase of the patient based on the identified sensor signal exhibiting the greatest confidence factor.

9.  The device of claim 1, further comprising:
a filter configured to filter the sensor signal such that zero crossings of the sensor signal indicate the midpoints of each respiratory phase.

10. The device of claim 9, wherein the filter comprises a bandpass filter.

11. The device of claim 1, further comprising:

an electrode to deliver electrical stimulation to an upper airway patency-related nerve of the patient,
wherein the control portion is configured to apply the electrical stimulation based on the predicted midpoint of the future inspiratory phase.

12. The device of claim 11, wherein the control portion is configured to apply the electrical stimulation starting relative to the predicted midpoint by a first predetermined interval and ending after the predicted midpoint by a second predetermined interval.

13. The device of claim 11, wherein the control portion is configured to apply the electrical stimulation starting an amount of time prior to the predicted midpoint computed as a percentage of a respiratory rate and ending after the predicted midpoint.

14. The device of claim 11, wherein the control portion is configured to apply the electrical stimulation starting an amount of time prior to the predicted midpoint computed as a percentage of a respiratory rate plus a predetermined amount of time and ending after the predicted midpoint.

15. The device of claim 1, wherein the control portion is configured to compute a respiration period as the midpoint of an inspiratory phase minus the midpoint of an immediately previous inspiratory phase.

**Patentansprüche**

1.  Vorrichtung, umfassend:

einen Sensor, der an einem Patienten befestigt werden kann, um ein Sensorsignal bereitzustellen, wobei der Sensor Änderungen der Beschleunigung erfasst, die für die Atmung des Patienten indikativ sind; und
einen Steuerungsabschnitt, der konfiguriert ist zum:

Bestimmen eines Mittelpunkts jeder Atemphase des Patienten basierend auf dem Sensorsignal;
Identifizieren jeder exspiratorischen Phase oder jeder inspiratorischen Phase des Patienten basierend auf dem Sensorsignal; und
Vorhersagen eines Mittelpunkts einer zukünftigen inspiratorischen Phase basierend auf vorherigen Mittelpunkten jeder Atemphase.

2.  Vorrichtung nach Anspruch 1, wobei der Steuerungsabschnitt konfiguriert ist, um zu identifizieren, ob die Steigung jeder exspiratorischen Phase positiv oder negativ ist.

3.  Vorrichtung nach Anspruch 2, wobei der Steuerungsabschnitt konfiguriert ist zum:

Bestimmen des Medians von Datenpunkten des Sensorsignals zwischen einem aktuellen Mittelpunkt und mindestens zwei vorherigen Mittelpunkten;
Identifizieren der Steigung der exspiratorischen Phase als negativ als Reaktion darauf, dass der Median kleiner als 0 ist; und
Identifizieren der Steigung der exspiratorischen Phase als positiv, als Reaktion darauf, dass der Median größer als 0 ist;

4.  Vorrichtung nach Anspruch 1, wobei der Steuerungsabschnitt konfiguriert ist, um zu identifizieren, ob die Neigung jeder inspiratorischen Phase positiv oder negativ ist.

**5.** Vorrichtung nach Anspruch 4, wobei der Steuerungsabschnitt konfiguriert ist zum:

Bestimmen des Medians von Datenpunkten des Sensorsignals zwischen einem aktuellen Mittelpunkt und mindestens zwei vorherigen Mittelpunkten;
Identifizieren der Steigung der inspiratorischen Phase als positiv als Reaktion darauf, dass der Median kleiner als 0 ist; und
Identifizieren der Steigung der inspiratorischen Phase als negativ als Reaktion darauf, dass der Median größer als 0 ist.

**6.** Vorrichtung nach Anspruch 1, wobei der Sensor einen Beschleunigungsmesser umfasst, um eine Rotationsbewegung zu erfassen.

**7.** Vorrichtung nach Anspruch 6, wobei der Beschleunigungsmesser einen dreiachsigen Beschleunigungsmesser umfasst.

**8.** Vorrichtung nach Anspruch 7, wobei der dreiachsige Beschleunigungsmesser drei entsprechende Sensorsignale bereitstellt, und
wobei der Steuerungsabschnitt konfiguriert ist zum:

Bestimmen eines Vertrauensfaktors, der jedem der drei jeweiligen Sensorsignale zugeordnet ist;
Identifizieren, welches jeweilige Sensorsignal den größten Vertrauensfaktor aufzeigt; und
Bestimmen des Mittelpunkts jeder Atemphase des Patienten basierend auf dem identifizierten Sensorsignal, das den größten Vertrauensfaktor aufzeigt.

**9.** Vorrichtung nach Anspruch 1, ferner umfassend:
einen Filter, der konfiguriert ist, um das Sensorsignal derart zu filtern, dass Nulldurchgänge des Sensorsignals bezeichnend für die Mittelpunkte jeder Atemphase sind.

**10.** Vorrichtung nach Anspruch 9, wobei der Filter einen Bandpassfilter umfasst.

**11.** Vorrichtung nach Anspruch 1, ferner umfassend:

eine Elektrode, um eine elektrische Stimulation an einen mit der Durchgängigkeit der oberen Atemwege in Zusammenhang stehenden Nerv des Patienten abzugeben,
wobei der Steuerungsabschnitt konfiguriert ist, um die elektrische Stimulation basierend auf dem vorhergesagten Mittelpunkt der zukünftigen inspiratorischen Phase anzuwenden.

**12.** Vorrichtung nach Anspruch 11, wobei der Steuerungsabschnitt konfiguriert ist, um die elektrische Stimulation beginnend relativ zu dem vorhergesagten Mittelpunkt um ein erstes vorbestimmtes Intervall und endend nach dem vorhergesagten Mittelpunkt um ein zweites vorbestimmtes Intervall anzuwenden.

**13.** Vorrichtung nach Anspruch 11, wobei der Steuerungsabschnitt konfiguriert ist, um die elektrische Stimulation beginnend eine Zeitspanne vor dem vorhergesagten Mittelpunkt, die als ein Prozentsatz einer Atemfrequenz berechnet wird, und endend nach dem vorhergesagten Mittelpunkt, anzuwenden.

**14.** Vorrichtung nach Anspruch 11, wobei der Steuerungsabschnitt konfiguriert ist, um die elektrische Stimulation beginnend eine Zeitspanne vor dem vorhergesagten Mittelpunkt, die als ein Prozentsatz einer Atemfrequenz plus einer vorbestimmten Zeitspanne berechnet wird, und endend nach dem vorhergesagten Mittelpunkt, anzuwenden.

**15.** Vorrichtung nach Anspruch 1, wobei der Steuerungsabschnitt konfiguriert ist, um eine Atmungsperiode als den Mittelpunkt einer inspiratorischen Phase minus den Mittelpunkt einer unmittelbar vorherigen inspiratorischen Phase zu berechnen.

**Revendications**

**1.** Dispositif comprenant :

un capteur pouvant être fixé sur un patient pour fournir un signal de capteur, le capteur étant destiné à détecter des changements d'accélération indiquant une respiration du patient ; et

une partie de commande configurée pour :

déterminer un point médian de chaque phase respiratoire du patient sur la base du signal de capteur ;

identifier chaque phase expiratoire ou chaque phase inspiratoire du patient sur la base du signal de capteur ; et

prédire un point médian d'une future phase inspiratoire sur la base de points médians précédents de chaque phase respiratoire.

2. Dispositif selon la revendication 1, dans lequel la partie de commande est configurée pour effectuer une identification précisant si la pente de chaque phase expiratoire est positive ou négative.

3. Dispositif selon la revendication 2, dans lequel la partie de commande est configurée pour :

déterminer la médiane de points de données du signal de capteur entre un point médian actuel et au moins deux points médians précédents ;

identifier la pente de la phase expiratoire comme étant négative lorsque la médiane est inférieure à 0 ; et

identifier la pente de la phase expiratoire comme étant positive lorsque la médiane est supérieure à 0.

4. Dispositif selon la revendication 1, dans lequel la partie de commande est configurée pour effectuer une identification précisant si la pente de chaque phase inspiratoire est positive ou négative.

5. Dispositif selon la revendication 4, dans lequel la partie de commande est configurée pour :

déterminer la médiane de points de données du signal de capteur entre un point médian actuel et au moins deux points médians précédents ;

identifier la pente de la phase inspiratoire comme étant positive lorsque la médiane est inférieure à 0 ; et

identifier la pente de la phase inspiratoire comme étant négative lorsque la médiane est supérieure à 0.

6. Dispositif selon la revendication 1, dans lequel le capteur comprend un accéléromètre pour détecter un mouvement de rotation.

7. Dispositif selon la revendication 6, dans lequel l'accéléromètre comprend un accéléromètre à trois axes.

8. Dispositif selon la revendication 7, dans lequel l'accéléromètre à trois axes fournit trois signaux de capteur respectifs, et

dans lequel la partie de commande est configurée pour :

déterminer un facteur de confiance associé à chacun des trois signaux de capteur respectifs ;

identifier quel signal de capteur respectif présente le facteur de confiance le plus élevé ; et

déterminer le point médian de chaque phase respiratoire du patient sur la base du signal de capteur identifié présentant le facteur de confiance le plus élevé.

9. Dispositif selon la revendication 1, comprenant en outre :
un filtre configuré pour filtrer le signal de capteur de sorte que les passages à zéro du signal de capteur indiquent les points médians de chaque phase respiratoire.

10. Dispositif selon la revendication 9, dans lequel le filtre comprend un filtre passe-bande.

11. Dispositif selon la revendication 1, comprenant en outre :

une électrode pour délivrer une stimulation électrique à un nerf lié à la perméabilité des voies respiratoires supérieures du patient,

dans lequel la partie de commande est configurée pour appliquer la stimulation électrique sur la base du point médian prédit de la future phase inspiratoire.

12. Dispositif selon la revendication 11, dans lequel la partie de commande est configurée pour appliquer la stimulation électrique commençant par rapport au point médian prédit à un premier intervalle prédéterminé et se terminant à un deuxième intervalle prédéterminé après le point médian prédit.

13. Dispositif selon la revendication 11, dans lequel la partie de commande est configurée pour appliquer la stimulation électrique commençant à un délai avant le point médian prédit calculé en pourcentage d'une fréquence respiratoire et se terminant après le point médian prédit.

14. Dispositif selon la revendication 11, dans lequel la partie de commande est configurée pour appliquer la stimulation électrique commençant à un délai avant le point médian prédit calculé en pourcentage d'une fréquence respiratoire plus un délai prédéterminé et se terminant après le point médian prédit.

15. Dispositif selon la revendication 1, dans lequel la partie de commande est configurée pour calculer une période respiratoire comme étant le point médian d'une phase inspiratoire moins le point médian d'une phase inspiratoire immédiatement précédente.

10A

12A

SENSOR → CONTROL PORTION

14A

**FIG. 1A**

10B

14B

CONTROL PORTION

12B

SENSOR

SENSE CHANGES IN
ACCELERATION

16

EXTRACT FEATURE(S) OF EACH
RESPIRATORY PHASE

18

IDENTIFY EACH RESPIRATORY PHASE

20

DETERMINE A CONFIDENCE FACTOR
FOR EACH RESPIRATORY PHASE

22

IDENTIFY SLOPE OF EACH
RESPIRATORY PHASE

24

PREDICT FUTURE INSPIRATORY PHASE

26

APPLY STIMULATION

28

**FIG. 1B**

**FIG. 2**

100

B2

B1

104A

G

CHEST WALL

102A

**FIG. 4**

148 ($Y_{R2}$)

144B

144A

143

144B

($Y_{R2}$)

148

142

144A

148

146 ($Y_{R1}$)

144C

($Y_{R1}$) 146

$T_i$  $T_{EP}$  $T_{EA}$  $T_i$

R

**FIG. 3**

A

P

B2

102B

B1

$Y_{R2}$

Ω

$Y_{R1}$

122A

122B

104A

104B

120

124

NECK

CHEST WALL

102A

S

I

G

P1

FIG. 5A

**FIG. 5B**

FIG. 5C

FIG. 5D

FIG. 5E

FIG. 5F

**FIG. 5G**

**FIG. 5H**

**FIG. 5I**

158

158A

DETERMINE THE MEDIAN OF DATA POINTS OF THE SENSOR SIGNAL BETWEEN A CURRENT MIDPOINT AND AT LEAST TWO PREVIOUS MIDPOINTS

158B

IDENTIFY THE SLOPE OF THE EXPIRATORY PHASE AS NEGATIVE IN RESPONSE TO THE MEDIAN BEING LESS THAN 0

158C

IDENTIFY THE SLOPE OF THE EXPIRATORY PHASE AS POSITIVE IN RESPONSE TO THE MEDIAN BEING GREATER THAN 0

FIG. 6A

159

159A

DETERMINE THE MEDIAN OF DATA POINTS OF THE SENSOR SIGNAL BETWEEN A CURRENT MIDPOINT AND AT LEAST TWO PREVIOUS MIDPOINTS

159B

IDENTIFY THE SLOPE OF THE INSPIRATORY PHASE AS POSITIVE IN RESPONSE TO THE MEDIAN BEING LESS THAN 0

159C

IDENTIFY THE SLOPE OF THE INSPIRATORY PHASE AS NEGATIVE IN RESPONSE TO THE MEDIAN BEING GREATER THAN 0

FIG. 6B

**FIG. 7A**

**FIG. 7B**

**FIG. 8**

**FIG. 9A**

**FIG. 9B**

**FIG. 9C**

**FIG. 10**

EP 4 351 417 B1

**FIG. 11**

**FIG. 12**

**FIG. 13**

500

ARRANGING THE ACCELERATION SENSOR AS N NUMBER OF ORTHOGONALLY-ARRANGED SINGLE AXIS ACCELERATION SENSING ELEMENTS

**FIG. 14A**

505

IDENTIFYING, VIA THE SENSING, WHICH OF THE N SINGLE AXIS ACCELERATION SENSING ELEMENTS EXHIBITS A REFERENCE ANGULAR ORIENTATION, DURING BREATHING, CLOSEST TO BEING GENERALLY PERPENDICULAR TO AN EARTH GRAVITY VECTOR

**FIG. 14B**

510

DETERMINING THE REFERENCE ANGULAR ORIENTATION OF EACH N AXIS ACCELERATION SENSING ELEMENTS AS AN INCLINATION ANGLE OF A MEASUREMENT AXIS OF EACH RESPECTIVE N AXIS ACCELERATION SENSING ELEMENTS RELATIVE TO THE GRAVITY VECTOR

**FIG. 14C**

520

IMPLEMENTING THE SENSING VIA SENSING AN AC SIGNAL COMPONENT OF THE RESPECTIVE ACCELERATION SENSING ELEMENTS WHILE EXCLUDING A DC SIGNAL COMPONENT OF THE RESPECTIVE ACCELERATION SENSING ELEMENTS

**FIG. 14D**

530

PERFORMING THE DETERMINATION OF RESPIRATION INFORMATION, VIA THE SENSED ROTATIONAL MOVEMENT, USING THE IDENTIFIED SENSING ELEMENT

**FIG. 14E**

535

SENSING AN AC SIGNAL COMPONENT OF THE IDENTIFIED SENSING ELEMENT WITHIN A RANGE OF ANGULAR ORIENTATIONS OF THE IDENTIFIED SENSING ELEMENT, WHEREIN A FIRST END OF THE RANGE OF ORIENTATIONS CORRESPONDS TO A PEAK EXPIRATION AND AN OPPOSITE SECOND END OF THE RANGE OF ORIENTATIONS CORRESPONDS TO A PEAK INSPIRATION

**FIG. 14F**

540

(1) IDENTIFYING WHICH OF THE N SINGLE AXIS ACCELERATION SENSING ELEMENTS EXHIBITS A REFERENCE ANGULAR ORIENTATION, DURING BREATHING, WITHIN A RANGE OF ABOUT 45 DEGREES TO ABOUT 135 DEGREES RELATIVE TO THE GRAVITY VECTOR; (2) SENSING, FOR EACH RESPECTIVE SENSING ELEMENT, A RANGE OF ANGULAR ORIENTATIONS WHEREIN A FIRST END OF THE RANGE CORRESPONDS TO PEAK EXPIRATION AND AN OPPOSITE SECOND END OF THE RANGE CORRESPONDS TO PEAK INSPIRATION; AND (3) DETERMINING WHICH OF THE RESPECTIVE SENSING ELEMENTS EXHIBITS A GREATEST RANGE OF ANGULAR ORIENTATIONS

**FIG. 14G**

545

PERFORMINING THE DETERMINATION OF RESPIRATION INFORMATION, VIA THE SENSED ROTATIONAL MOVEMENT, USING THE IDENTIFIED SENSING ELEMENT DETERMINED TO EXHIBIT THE GREATEST RANGE OF ANGULAR ORIENTATIONS

**FIG. 14H**

560

IDENTIFYING WHICH OF THE N SINGLE AXIS ACCELERATION SENSING ELEMENTS, DURING BREATHING, EXHIBITS A GREATEST RANGE OF VALUES OF AN AC SIGNAL COMPONENT

**FIG. 14I**

570

PERFORMINING THE DETERMINATION OF RESPIRATION INFORMATION, VIA THE SENSED ROTATIONAL MOVEMENT, USING THE IDENTIFIED SENSING ELEMENT DETERMINED TO EXHIBIT THE GREATEST RANGE OF VALUES OF THE AC SIGNAL COMPONENT

**FIG. 14J**

580

SENSING AN AC SIGNAL COMPONENT OF THE IDENTIFIED SENSING ELEMENT, DURING BREATHING, WHEREIN A FIRST END OF A RANGE OF VALUES OF THE SENSED AC SIGNAL COMPONENT CORRESPONDS TO A PEAK EXPIRATION AND AN OPPOSITE SECOND END OF THE RANGE OF VALUES OF THE AC SIGNAL COMPONENT CORRESPONDS TO A PEAK INSPIRATION

**FIG. 14K**

**FIG. 15**

**FIG. 16A**

**FIG. 16B**

**FIG. 16C**

**FIG. 16D**

**FIG. 16E**

EP 4 351 417 B1

FIG. 16F

*770*

SENSED ACCELERATION SIGNAL(S) — 710

FILTERING — 772

VIRTUAL VECTOR — 776

FEATURE EXTRACTION — 778

CONFIDENCE FACTOR — 781

QUALITY ASSESSMENT — 780

DEFAULT RESPIRATORY PHASE — 790

CONFIDENCE — 791

RESPIRATORY PHASE INFORMATION — 750

INSPIRATORY PHASE — 752

EXPIRATORY ACTIVE PHASE — 754

EXPIRATORY PAUSE PHASE — 756

RESPIRATORY PARAMETERS — 760

902 ⬊    <u>900</u>    ⬋ 910

| CONTROLLER |
| PROCESSOR |

| MEMORY |
| INSTRUCTIONS | ～911 |
| INFORMATION | ～912 |

904

**FIG. 17A**

<u>920</u>
CONTROL
PORTION

| PULSE GENERATOR | 925 |

930
| REMOTE | | PATIENT | 932 |
| | PHYSICIAN | 934 |

**FIG. 17B**

⬋ 940

| USER INTERFACE |
| INPUT | DISPLAY |

942

944

**FIG. 18**

1000

1002

SENSING ROTATIONAL MOVEMENT INDICATIVE OF RESPIRATION OF A
PATIENT TO GENERATE A SENSOR SIGNAL

1004

ANALYZING THE SENSOR SIGNAL TO IDENTIFY A FEATURE OF EACH
EXPIRATORY PHASE AND/OR EACH INSPIRATORY PHASE OF THE RESPIRATION
OF THE PATIENT

1006

CALCULATING A PREDICTED FEATURE OF A FUTURE INSPIRATORY PHASE
BASED ON PREVIOUS FEATURES OF EACH RESPIRATORY PHASE

1008

BEGINNING STIMULATION OF AN UPPER AIRWAY PATENCY-RELATED NERVE
OF THE PATIENT BASED ON THE PREDICTED FEATURE OF THE FUTURE
INSPIRATORY PHASE, AND CONTINUING STIMULATION OF THE UPPER
AIRWAY PATENCY-RELATED NERVE DURING AN INSPIRATORY PHASE
CORRESPONDING TO THE FUTURE INSPIRATORY PHASE

FIG. 19

1100

1102

SENSING ROTATIONAL MOVEMENT INDICATIVE OF RESPIRATION OF A
PATIENT TO GENERATE A SENSOR SIGNAL

1104

ANALYZING THE SENSOR SIGNAL TO IDENTIFY A MIDPOINT OF EACH
EXPIRATORY PHASE AND EACH INSPIRATORY PHASE OF THE RESPIRATION OF
THE PATIENT

1106

CALCULATING A PREDICTED MIDPOINT OF A FUTURE INSPIRATORY PHASE
BASED ON PREVIOUS MIDPOINTS OF EACH RESPIRATORY PHASE

1108

BEGINNING STIMULATION OF AN UPPER AIRWAY PATENCY-RELATED NERVE
OF THE PATIENT A PREDETERMINED AMOUNT OF TIME PRIOR TO THE
CALCULATED MIDPOINT OF THE FUTURE INSPIRATORY PHASE, AND
CONTINUING STIMULATION OF THE UPPER AIRWAY PATENCY-RELATED
NERVE DURING AN INSPIRATORY PHASE CORRESPONDING TO THE FUTURE
INSPIRATORY PHASE

FIG. 20

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8938299 B
- US 20180117316 A, Wagner
- US 5944680 A, Christopherson
- US 5522862 A
- US 10583297 B, Ni
- US 8340785 B, Bonde
- US 9227053 B, Bonde
- US 8934992 B, Johnson
- WO 2019032890 A, Rondoni
- US 48595419
- US 6572543 B, Christopherson
- US 9889299 B, Ni
- US 9486628 B, Christopherson